# EUROPEAN PATENT APPLICATION

(11) **EP 4 796 551 A1**
(43) Date of publication of application: **26.08.2026**
(21) Application number: 24879739.1
(22) Date of filing: 16.10.2024
(51) Int. Cl.: C07F 9/09, A61K 9/127, A61K 47/24, A61K 47/28, A61K 47/34, A61K 48/00

(54) **ZWITTERIONIC PHOSPHOLIPID COMPOUND AND LIPID NANOPARTICLE CONTAINING THE SAME**

(30) Priority: 16.10.2023 JP 2023178361
(71) Applicant: National University Corporation Hokkaido University, Hokkaido 060-0808 (JP)
(72) Inventor: SATO, Yusuke, Sapporo-shi, Hokkaido 060-0808 (JP); HARASHIMA, Hideyoshi, Sapporo-shi, Hokkaido 060-0808 (JP)
(74) Representative: Müller-Boré & Partner Patentanwälte PartG mbB
(86) International application number: PCT/JP2024/036819
(87) International publication number: WO 2025/084317

(57) **Abstract**

The purpose of the invention described herein is to provide a compound having a novel chemical structure that is usable for lipid nanoparticles. Disclosed are a compound represented by formula (I) (in the formula, R¹ and R² are each independently a C10-24 chain hydrocarbon group, R³ is a C1-22 chain hydrocarbon group) and lipid nanoparticles containing the compound.

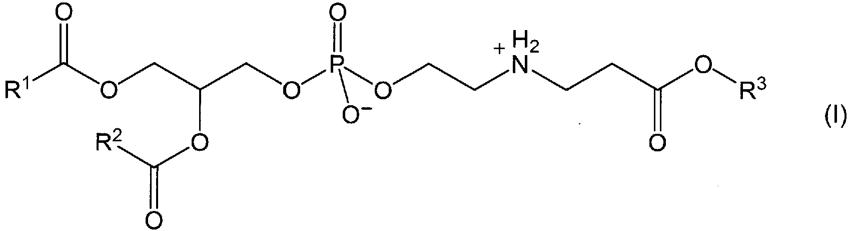

## Description

### Technical Field

The present invention relates to a zwitterionic phospholipid compound having a novel chemical structure and a lipid nanoparticle comprising the same.

### Background Art

Lipid nanoparticles (LNPs) are utilized as carriers for encapsulating lipophilic drugs and nucleic acids such as siRNA (short interfering RNA) and mRNA to deliver them to target cells. For example, Patent Literature 1 describes a lipid nanoparticle comprising as a constituent lipid a pH-sensitive cationic lipid that is electrically neutral at physiological pH and changes to cationic under a weakly acidic pH environment such as an endosome, as a carrier for efficiently delivering a nucleic acid such as siRNA into a target cell. In addition, Non-patent Literatures 1 to 4 also describe various pH-sensitive cationic lipids that can be utilized as constituent lipids of lipid nanoparticles, and Patent Literature 2 describes that a lipid nanoparticle comprising a pH-sensitive cationic lipid having a branched hydrocarbon chain as a constituent lipid has high selectivity to the liver or spleen.

Lipid nanoparticles are taken up into cells by endocytosis. Therefore, for improvement of uptake efficiency of lipid nanoparticles into cells, it is essential that membrane fusion between the lipid nanoparticle and an endosomal membrane readily occurs. On the other hand, phosphatidylcholine (PC), which is a main constituent lipid of the endosomal membrane, stabilizes a lamellar (L) phase of a lipid bilayer membrane (planar membrane) because it has a cylindrical molecular shape, and strongly suppresses transition to an inverted hexagonal (H_{II}) phase essential for membrane fusion. For example, it has long been known that 1,2-dioleoyl-sn-glycero-3-phosphoethanolamine (DOPE), which is a constituent lipid widely used for lipid nanoparticles, promotes transition from the lamellar phase to the inverted hexagonal phase because it has a cone-like molecular shape, whereas its action is significantly inhibited by PC (Non-patent Literature 5).

As a method for producing lipid nanoparticles encapsulating nucleic acids and the like, there is a method based on the principle of an alcohol dilution method using a flow path. For example, Non-patent Literature 6 describes that lipid nanoparticles having a diameter of about 30 nm can be reproducibly produced by using a microchannel with a built-in three-dimensional micromixer capable of achieving instantaneous mixing of two liquids. Further, Patent Literature 3 and Non-patent Literature 7 describe that by using a flow path structure having a simple two-dimensional structure in which baffles (baffle plates) having a constant width relative to the flow path width are arranged alternately from both sides in a micro-sized flow path through which a raw material solution flows, a nano-sized lipid particle formation system with higher particle size controllability than a flow path structure using a conventional three-dimensional mixer can be formed. These methods for producing nanoparticle formulations are mainly adopted for the production of lipid nanoparticles loaded with lipophilic drugs or nucleic acids such as siRNA or mRNA in recent years.

### Citation List

### Patent Literatures

Patent Literature 1: International Publication No. 2018/230710
Patent Literature 2: International Publication No. 2022/071582
Patent Literature 3: International Publication No. 2018/190423

### Non-patent Literatures

Non-patent Literature 1: Jayaraman et al., Angewandte Chemie International Edition, 2012, vol.51, p.8529-8533.
Non-patent Literature 2: Watanabe et al., Scientific Reports, 2014, 4:4750, DOI: 10.1038/srep04750.
Non-patent Literature 3: Yamamoto et al., Journal of Hepatology, 2016, vol.64, p.547-555.
Non-patent Literature 4: Sato et al., Molecular Therapy, 2016, vol.24, p.788-795.
Non-patent Literature 5: Cullis et al., Biochimica et Biophysica Acta, 1979, vol.559, p.399-420
Non-patent Literature 6: Leung et al., Journal of Physical Chemistry C Nanomater Interfaces, 2012, vol.116(34), p.18440-18450
Non-patent Literature 7: N. Kimura, et al., Acs Omega 3 (2018) 5044-5051, https://doi.org/10.1021/acsomega.8b00341

### Summary of Invention

For efficient substance delivery by lipid nanoparticles comprising a conventional pH-sensitive cationic lipid, it is a premise that the pH-sensitive cationic lipid is sufficiently positively charged in response to pH reduction; anionic lipids interacting therewith are present sufficiently in the endosomal membrane; and phase transition driven by cationic lipid-anionic lipid interaction is not inhibited by neutral phospholipids (such as phosphatidylcholine); however, if at least a part of these conditions is not satisfied, efficient substance delivery cannot be expected; thus, a phase transition means by a different mechanism has been demanded. Accordingly, an object of the present invention is to provide a compound having a novel chemical structure usable for a lipid nanoparticle.

As a result of intensive studies to solve the above problems, the present inventors have found that a zwitterionic phospholipid compound having a novel chemical structure obtained as a result of search based on the chemical structure of DOPE is suitable as a constituent lipid of a lipid nanoparticle, and have completed the present invention. That is, the present invention provides the following compounds, lipid nanoparticles comprising the same, and compositions comprising the same.
[1] A compound represented by formula (I): 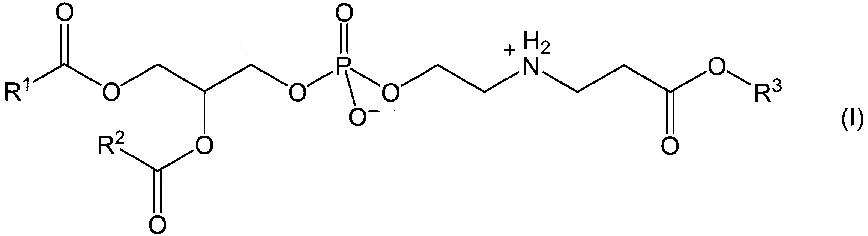 wherein
   R¹ and R² are each independently a chain hydrocarbon group having 10 to 24 carbon atoms, and
   R³ is a chain hydrocarbon group having 1 to 22 carbon atoms.
[2] The compound according to the above [1], wherein R¹ and/or R² is an unsaturated hydrocarbon group.
[3] The compound according to the above [1] or [2], wherein R¹ and/or R² is an alkenyl group, and/or
   R³ is an alkyl group or an alkenyl group.
[4] The compound according to any one of the above [1] to [3], wherein the number of carbon atoms of R¹ is 12 to 22, and/or
   the number of carbon atoms of R² is 12 to 22, and/or
   the number of carbon atoms of R³ is 2 to 20.
[5] The compound according to any one of the above [1] to [4], wherein R¹ and/or R² is 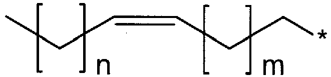 wherein
   an asterisk represents a binding site,
   n is an integer of 5 to 9, m is an integer of 2 to 8, provided that n+m is 12 to 16,
      and/or
   R³ is 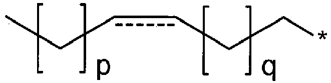 wherein
      an asterisk represents a binding site,
      p is an integer of 0 to 16, q is an integer of 0 to 16, provided that p+q is 0 to 16, and
      a double line composed of a solid line and a dashed line means a single bond or a double bond.
[6] The compound according to any one of the above [1] to [4], wherein R¹ and/or R² is 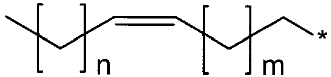 wherein
   an asterisk represents a binding site,
   n is an integer of 5 to 9, m is an integer of 2 to 8, provided that n+m is 12 to 16,
      and/or
   R³ is 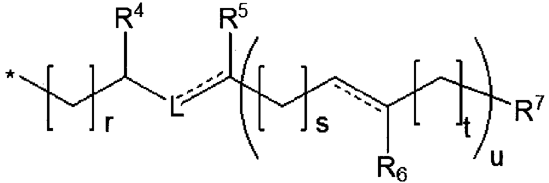 wherein
      an asterisk represents a binding site,
      R⁴ is a hydrogen atom or an alkyl group having 1 to 6 carbon atoms,
      R⁵ is a hydrogen atom or an alkyl group having 1 to 6 carbon atoms,
      R⁶ is a hydrogen atom or an alkyl group having 1 to 6 carbon atoms,
      provided that at least one of R⁴, R⁵, and R⁶ is an alkyl group having 1 to 6 carbon atoms,
      L is a single bond, an alkylene group having 1 to 4 carbon atoms, -CH=CH(CH₂)₂-, -CH₂CH=CHCH₂-, or -CH=CHCH₂-,
      R⁷ is a hydrogen atom or a methyl group,
      r is an integer of 0 to 4,
      s is an integer of 0 to 4,
      t is an integer of 0 to 4,
      u is 0 or 1,
      double lines composed of a solid line and a dashed line each independently mean a single bond or a double bond.
[7] The compound according to any one of the above [1] to [6], having any of the following structures: 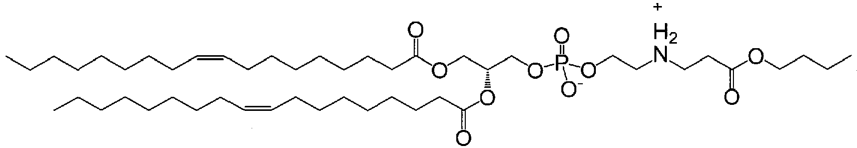 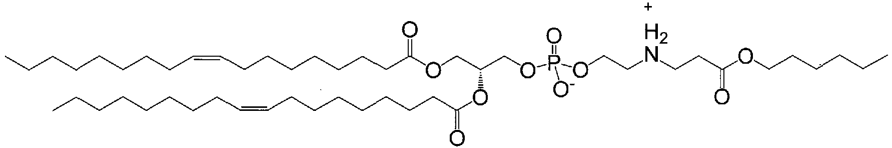 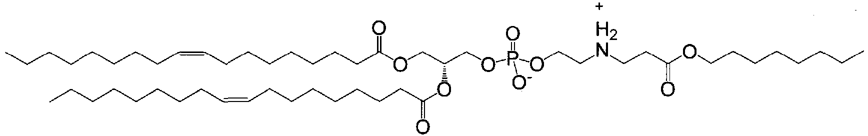 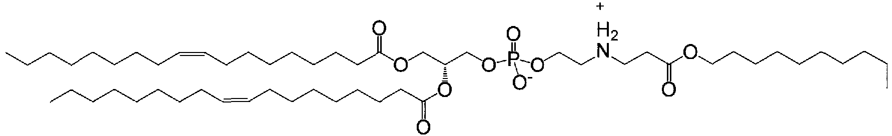 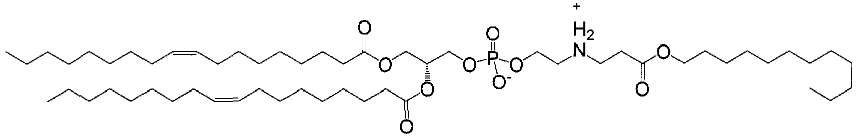 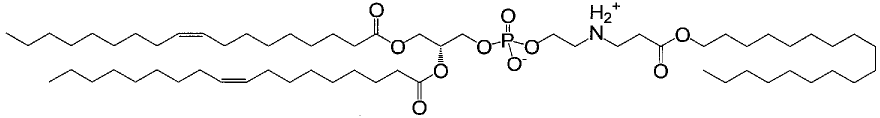 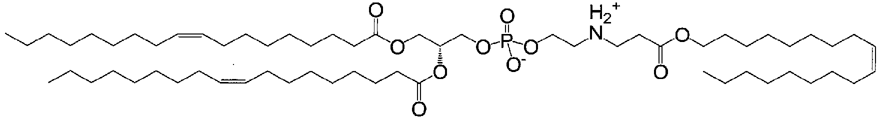 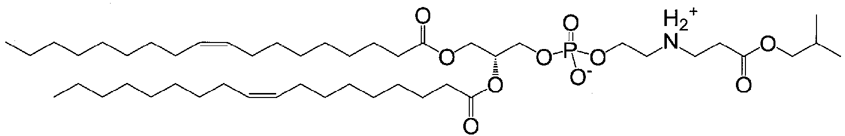 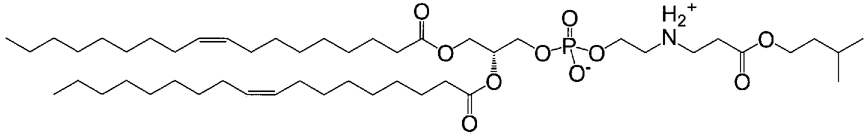 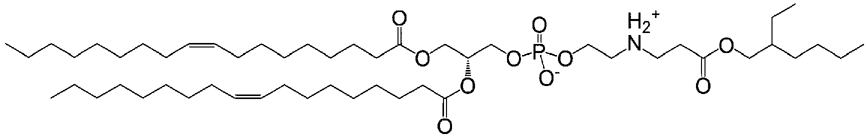
[8] The compound according to any one of the above [1] to [4] and [6], having any of the following structures: 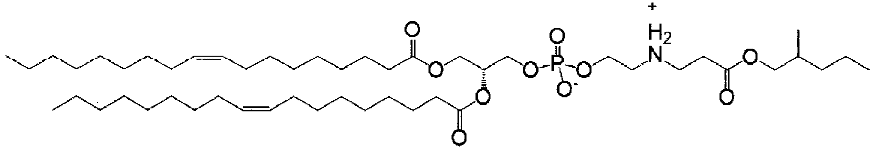 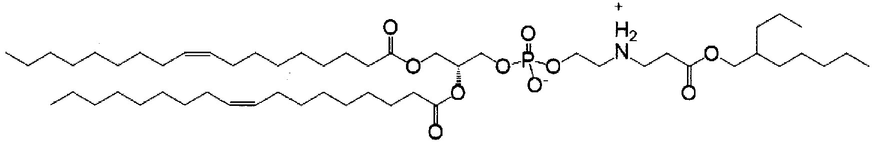 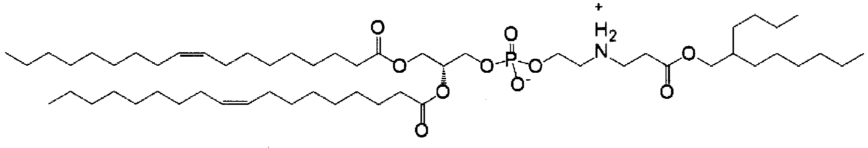 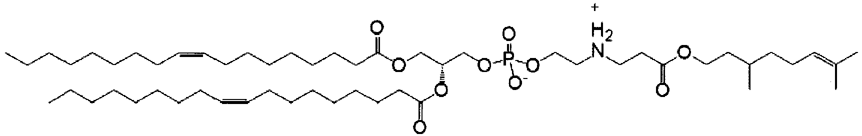 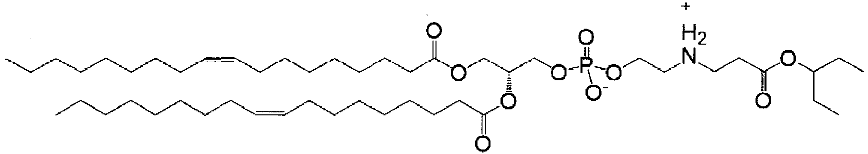 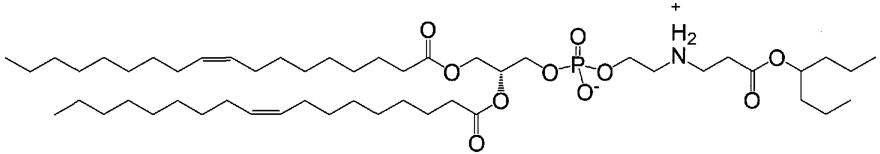
[9] A method for producing the compound according to any one of the above [1] to [8], comprising a step of adding, to a compound represented by formula (II): 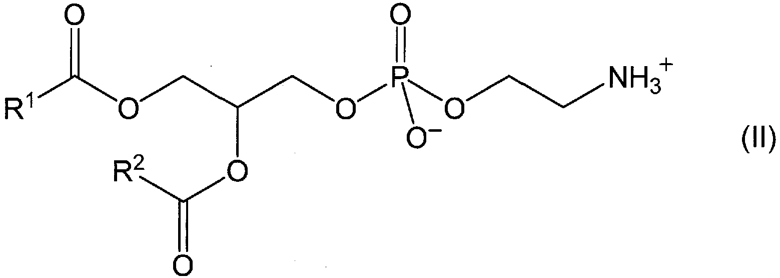
   wherein R¹ and R² are as defined in the above [1],
   a compound represented by formula (III): 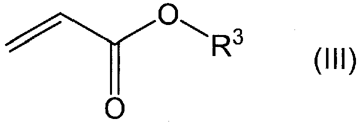
   wherein R³ is as defined in the above [1].
[10] A lipid nanoparticle comprising the compound according to any one of the above [1] to [8].
[11] The lipid nanoparticle according to the above [10], further comprising a sterol and/or a polyalkylene glycol-modified lipid.
[12] The lipid nanoparticle according to the above [10] or [11], further comprising a pH-sensitive cationic lipid having a pKa of 6.6 or less.
[13] The lipid nanoparticle according to any one of the above [10] to [12], further comprising a nucleic acid.
[14] The lipid nanoparticle according to the above [13], wherein the nucleic acid comprises siRNA, mRNA, and/or plasmid DNA.
[15] A composition comprising the lipid nanoparticle according to any one of the above [10] to [14].
[16] The composition according to the above [15], wherein the lipid nanoparticle comprises a nucleic acid.
[17] The composition according to the above [15] or [16], for expressing a foreign gene and/or for use in gene therapy.

According to the present invention, the compound represented by the formula (I) (also referred to herein as "DOPE-Cx") useful as a constituent lipid of a lipid nanoparticle is provided. Using the compound as a constituent lipid of a lipid nanoparticle enables efficient substance delivery by the lipid nanoparticle.

### Brief Description of Drawings

Fig. 1 shows an image diagram considered as an example of a mechanism of membrane fusion promotion between a lipid nanoparticle using the compound represented by the formula (I) and an endosome. (LNP means lipid nanoparticle; DSPC means distearoylphosphatidylcholine; DOPE-Cx means the compound represented by the formula (I); CL means cardiolipin; BMP (LBPA) means lysobisphosphatidic acid; and PC means phosphatidylcholine.)
Fig. 2 shows a graph of luciferase activity in the liver after administration of lipid nanoparticles comprising various phospholipids.
Fig. 3 shows a graph of luciferase activity in the liver after administration of lipid nanoparticles comprising various phospholipids.
Fig. 4 shows a graph of luciferase activity in the liver after administration of lipid nanoparticles comprising various phospholipids.
Fig. 5 shows a fluorescence photograph observing expression of EGFP in the liver after administration of lipid nanoparticles comprising various phospholipids.
Fig. 6A shows a graph of hemolytic activity at pH 5.0 by lipid nanoparticles comprising various phospholipids.
Fig. 6B shows a graph of hemolytic activity at pH 6.0 by lipid nanoparticles comprising various phospholipids.
Fig. 6C shows a graph of hemolytic activity at pH 7.4 by lipid nanoparticles comprising various phospholipids.
Fig. 7A shows results of measurement by a ³¹P NMR method when DOPE and POPC were mixed at a predetermined ratio.
Fig. 7B shows results of measurement by a ³¹P NMR method when DOPE-C8 and POPC were mixed at a predetermined ratio.
Fig. 8A shows results of measurement by a ³¹P NMR method when various DOPE-Cx and POPC were mixed at a ratio of 1:1.
Fig. 8B shows results of measurement by a ³¹P NMR method when various DOPE-Cx and POPC were mixed at a ratio of 1:1.
Fig. 9 shows results of measurement by a ³¹P NMR method when various DOPE-Cx and POPC were mixed at a ratio of 1:1.
Fig. 10 shows results of measurement by a SAXS method when DOPE-C8 and POPC were mixed at a ratio of 1:1.

### Description of Embodiments

Hereinafter, the present invention will be described in more detail.

The present invention relates to a compound represented by formula (I): 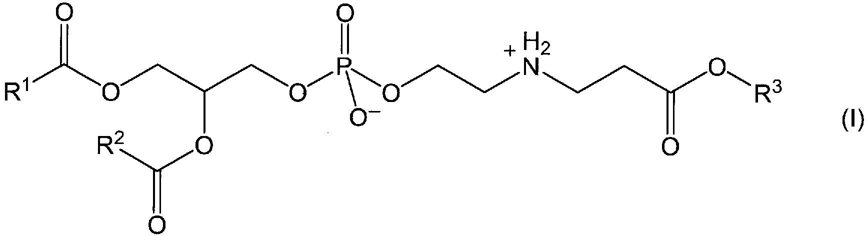 wherein
R¹ and R² are each independently a chain hydrocarbon group having 10 to 24 carbon atoms, and
R³ is a chain hydrocarbon group having 1 to 22 carbon atoms.

The compound of the present invention is a zwitterionic phospholipid compound and is designed to actively interact with phosphatidylcholine (PC), which is a neutral phospholipid serving as a major cell membrane constituent lipid, to promote membrane fusion. In the formula (I), one asymmetric carbon exists, and the compound may include an R-isomer or an S-isomer, but is preferably the R-isomer.

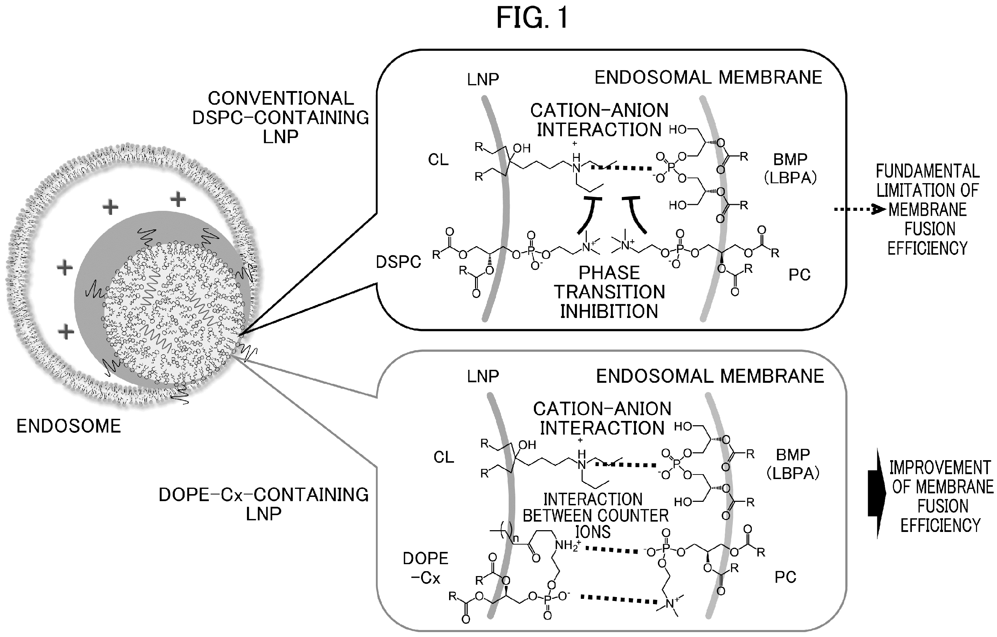

Conventional ionizable lipids form a counter ion by electrostatically interacting with an anionic lipid present in an endosomal membrane at a weakly acidic pH, and induce membrane fusion by promoting phase transition from a lamellar phase (planar membrane) inherent to a cell membrane to a non-lamellar phase having a high negative curvature. Since PC occupying a large part of the cell membrane has a cylindrical (columnar) molecular shape and is easily aligned on a plane, it stabilizes the lamellar phase and inhibits transition to the non-lamellar phase. Since positively charged ionizable lipids cannot electrostatically neutralize PC which is a neutral phospholipid, it is considered that they are susceptible to an inhibitory effect on phase transition by the neutral phospholipid (upper center of Fig. 1). Therefore, it is expected that incorporating a functional lipid that spontaneously interacts with PC to promote phase transition to the non-lamellar phase into a lipid nanoparticle promotes fusion between the lipid nanoparticle and the endosomal membrane, thereby realizing efficient substance delivery (such as mRNA introduction) into a cell.

Although not bound by a particular theory, for example, the net charge of PC is neutral, but a phosphocholine group which is a hydrophilic group possessed by PC has a zwitterionic structure composed of one anionic group (phosphate group) and one cationic group (choline group); since the anionic group is oriented on the side closer to a hydrophobic scaffold and the cationic group is oriented on the side farther therefrom, it is considered that a lipid having a zwitterionic structure with an orientation opposite to or different from that of the phosphocholine group relative to the hydrophobic scaffold causes electrostatic interaction between mutual zwitterions when coming into contact with PC. The compound of the present invention has a structure similar to DOPE whose phase transition activity is inhibited by PC, but in the compound of the present invention, the orientation of charged groups is changed by linking a hydrophobic group to the side opposite to the phosphate group in the choline group moiety, whereby it is considered that interaction between counter ions occurs between the compound of the present invention and PC to promote fusion between the lipid nanoparticle and the endosomal membrane (lower center of Fig. 1).

The "chain hydrocarbon group" described herein refers to a hydrocarbon group in which carbon atoms constituting a main chain are arranged linearly without having a cyclic structure. The chain hydrocarbon group may be a saturated hydrocarbon group or an unsaturated hydrocarbon group. Further, carbon atoms of the chain hydrocarbon group may be linked linearly without branching (straight chain) or may be linked with branching (branched chain).

In a certain embodiment, R¹, which is a chain hydrocarbon group that may be straight or branched, may be an unsaturated hydrocarbon group such as an alkenyl group. Further, the number of carbon atoms of R¹ is not particularly limited as long as it is within the range of 10 to 24, but may be, for example, 12 to 22 or 14 to 20. Specifically, R¹ may be 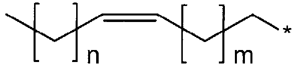 wherein
an asterisk represents a binding site,
n is an integer of about 5 to about 9, m is an integer of about 2 to about 8, provided that n+m is about 12 to about 16.

In a certain embodiment, R², which is a chain hydrocarbon group that may be straight or branched, may be an unsaturated hydrocarbon group such as an alkenyl group. Further, the number of carbon atoms of R² is not particularly limited as long as it is within the range of 10 to 24, but may be, for example, 12 to 22 or 14 to 20. Specifically, R² may be 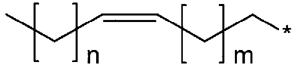 wherein
an asterisk represents a binding site,
n is an integer of about 5 to about 9, m is an integer of about 2 to about 8, provided that n+m is about 12 to about 16.

In a certain embodiment, R³, which is a chain hydrocarbon group that may be straight or branched, may be a saturated hydrocarbon group such as an alkyl group or an unsaturated hydrocarbon group such as an alkenyl group. Further, the number of carbon atoms of R³ is not particularly limited as long as it is within the range of 1 to 22, but may be, for example, 2 to 20 or 3 to 18 or 4 to 12. It is more preferable for efficient substance delivery, for example, intracellular delivery of a nucleic acid such as mRNA, that the number of carbon atoms of R³ is within the range of 5 to 8. Specifically, R³ may be 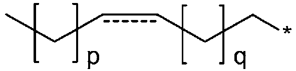 wherein
an asterisk represents a binding site,
p is an integer of about 0 to about 16, q is an integer of about 0 to 16, provided that p+q is about 0 to about 16, and
a double line composed of a solid line and a dashed line means a single bond or a double bond,
or may be 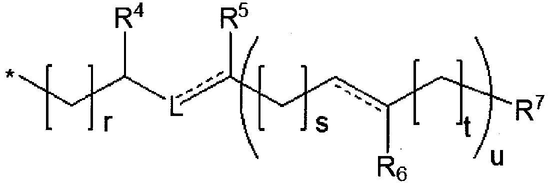 wherein
   an asterisk represents a binding site,
   R⁴ is a hydrogen atom, or an alkyl group having 1 to 6, 1 to 5, 1 to 4, 1 to 3, or 1 to 2 carbon atoms,
   R⁵ is a hydrogen atom or an alkyl group having 1 to 6, 1 to 5, 1 to 4, 1 to 3, or 1 to 2 carbon atoms,
   R⁶ is a hydrogen atom or an alkyl group having 1 to 6, 1 to 5, 1 to 4, 1 to 3, or 1 to 2 carbon atoms,
   provided that at least one of R⁴, R⁵, and R⁶ is an alkyl group having 1 to 6, 1 to 5, 1 to 4, 1 to 3, or 1 to 2 carbon atoms,
   L is a single bond, an alkylene group having 1 to 4, 1 to 3, or 1 to 2 carbon atoms, -CH=CH(CH₂)₂-, -CH₂CH=CHCH₂-, or -CH=CHCH₂-,
   R⁷ is a hydrogen atom or a methyl group,
   r is an integer of 0 to about 4,
   s is an integer of 0 to about 4,
   t is an integer of 0 to about 4,
   u is 0 or 1,
   double lines composed of a solid line and a dashed line each independently mean a single bond or a double bond.

Specific embodiments of the compound of the present invention are not particularly limited, but examples thereof include compounds having the following structures.

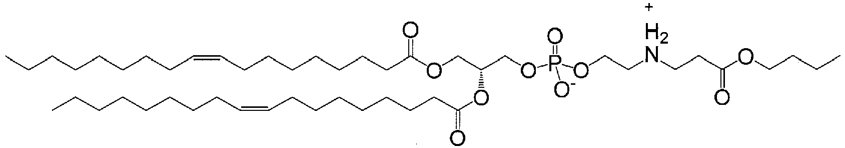

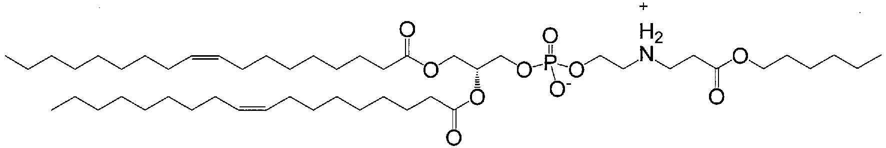

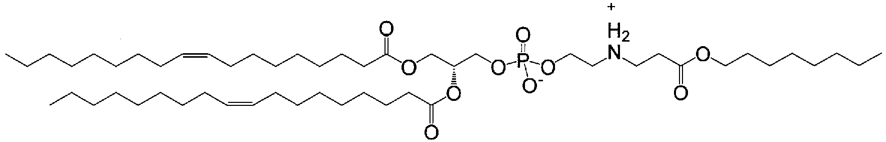

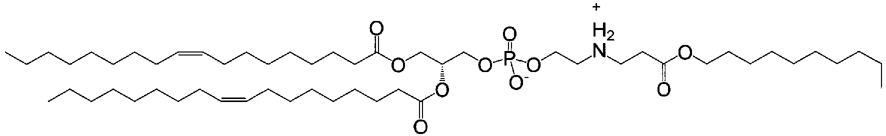

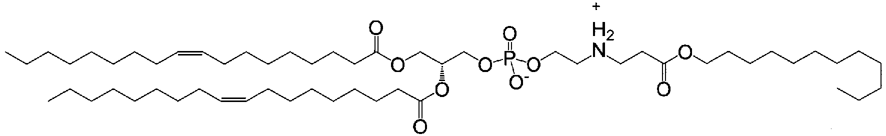

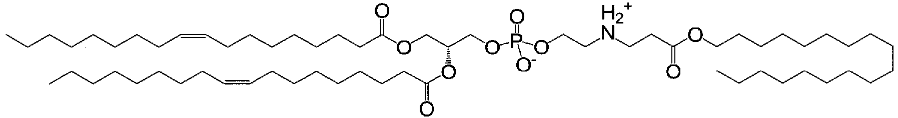

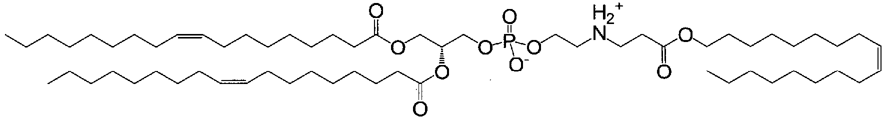

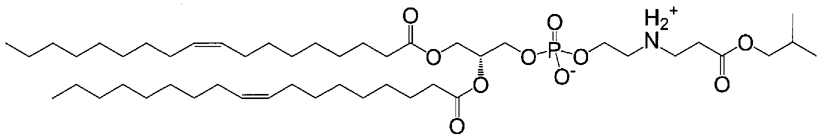

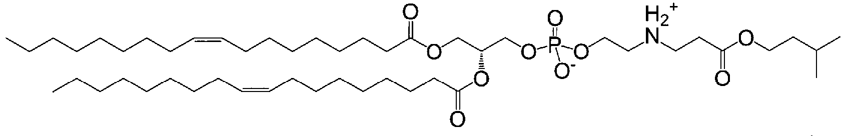

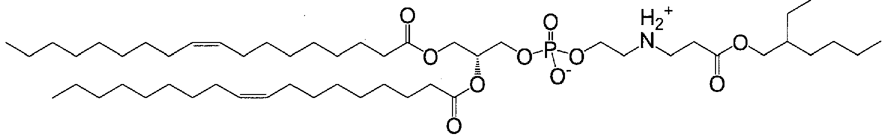

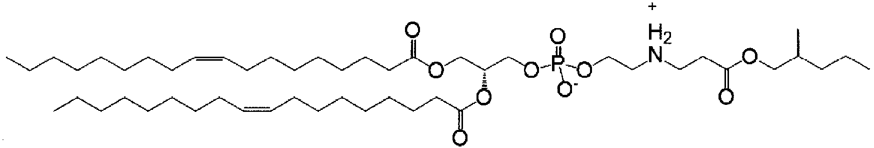

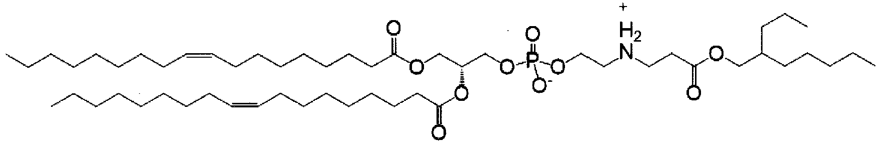

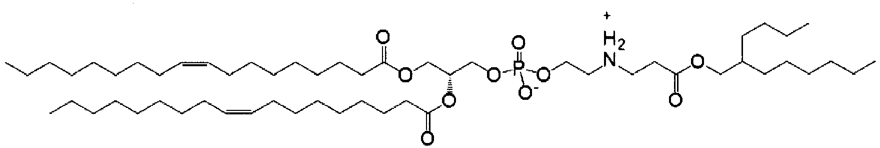

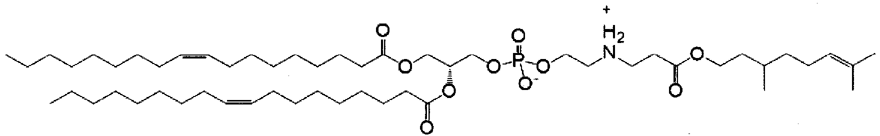

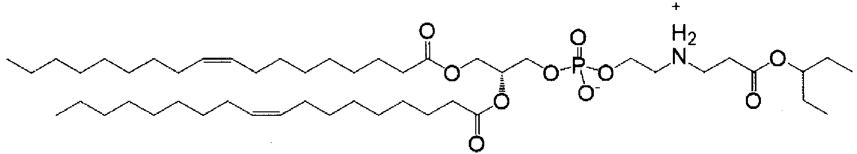

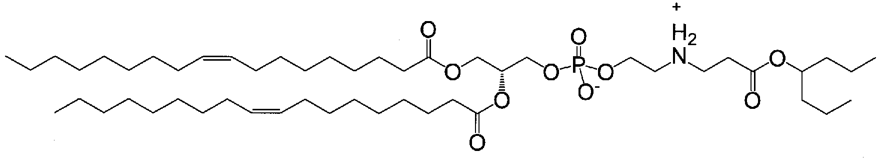

A method for synthesizing the compound of the present invention is not particularly limited, and the compound can be appropriately synthesized using a chemical reaction commonly used in the art. For example, the compound of the present invention may be produced by adding, to a compound represented by formula (II): 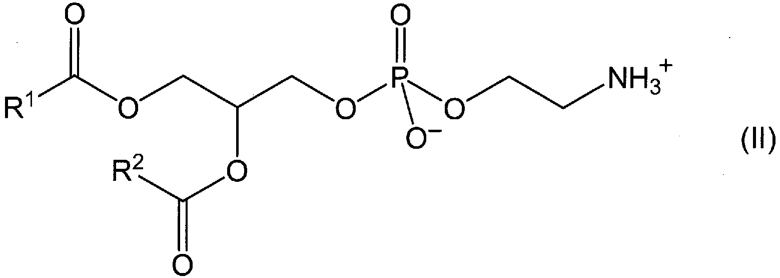
wherein R¹ and R² are as described above in relation to the compound of the present invention,
   a compound represented by formula (III): 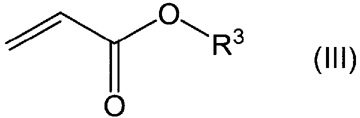
wherein R³ is as described above in relation to the compound of the present invention. That is, in another embodiment, the present invention also relates to a method for producing the compound represented by the formula (I), and the method comprises a step of adding the compound represented by the formula (III) to the compound represented by the formula (II). As a reaction used in the addition step, a reaction commonly used in the art can be adopted without particular limitation, but for example, the compound represented by the formula (III) may be added to the compound represented by the formula (II) by a Michael addition reaction.

In another embodiment, the present invention relates to a lipid nanoparticle comprising the compound represented by the formula (I) as a constituent lipid. The compound represented by the formula (I) is as described above as one embodiment of the present invention. The lipid nanoparticle of the present invention may comprise only one kind or two or more kinds of the compound represented by the formula (I).

The proportion of the compound represented by the formula (I) with respect to the total lipid amount in the lipid nanoparticle of the present invention ([amount of the compound represented by the formula (I) (mol)]/([total amount of lipid constituting the lipid nanoparticle (mol)])×100%) is not particularly limited, but may be, for example, about 1 mol% or more, preferably about 3 mol% or more, and more preferably about 5 mol% or more. The proportion of the compound represented by the formula (I) with respect to the total lipid amount in the lipid nanoparticle of the present invention may also be about 90 mol% or less, preferably about 80 mol% or less, and more preferably about 70 mol% or less. In a certain embodiment, the proportion of the compound represented by the formula (I) with respect to the total lipid amount in the lipid nanoparticle of the present invention may also be about 50 mol% or less, preferably about 30 mol% or less, and more preferably about 5 to about 20 mol%. Using the compound represented by the formula (I) within those ranges makes it suitable for preparing lipid nanoparticles with desired properties when combined with lipids having other functions.

In a certain embodiment, the lipid nanoparticle of the present invention may further comprise a pH-sensitive cationic lipid as a constituent lipid. The pH-sensitive cationic lipid is not particularly limited, and known pH-sensitive cationic lipids and modified compounds thereof can be appropriately used. Examples of known pH-sensitive cationic lipids include pH-sensitive cationic lipids described in Non-patent Literature 1 such as DLin-MC3-DMA, pH-sensitive cationic lipids described in Non-patent Literatures 2 to 4 such as YSK05 and YSK13-C3, pH-sensitive cationic lipids described in Patent Literature 1 such as CL4H6, and pH-sensitive cationic lipids described in Patent Literature 2 such as CL4F6. A pKa of the pH-sensitive cationic lipid is not particularly limited, but may be, for example, about 6.6 or less, preferably about 5.8 to about 6.6, and more preferably about 6.0 to about 6.6.

In a certain embodiment, the lipid nanoparticle of the present invention may further comprise as a constituent lipid an additional lipid generally used in forming liposomes other than the compound represented by the formula (I) and the pH-sensitive cationic lipid. The additional lipids are not particularly limited, but examples thereof include phospholipids, sterols, glycolipids, and saturated or unsaturated fatty acids. These can be used alone or in combination of two or more. In a particular embodiment, the lipid nanoparticle of the present invention may further comprise a phospholipid and/or a sterol.

The phospholipids are not particularly limited but, examples thereof include glycerophospholipids such as phosphatidylserine, phosphatidylinositol, phosphatidylglycerol, phosphatidylethanolamine, phosphatidylcholine, cardiolipin, plasmalogen, ceramide phosphorylglycerol phosphate, and phosphatidic acid; and sphingophospholipids such as sphingomyelin, ceramide phosphorylglycerol, and ceramide phosphorylethanolamine. Naturally occurring phospholipids such as egg yolk lecithin and soybean lecithin can also be used. The fatty acid residues in the glycerophospholipids and sphingophospholipids are not particularly limited, but may be, for example, saturated or unsaturated fatty acid residues having about 12 to about 24 carbon atoms, and preferably saturated or unsaturated fatty acid residues having about 14 to about 20 carbon atoms. Specifically, the fatty acid residues in the glycerophospholipids and sphingophospholipids may be acyl groups derived from fatty acids such as lauric acid, myristic acid, palmitic acid, palmitoleic acid, stearic acid, oleic acid, linoleic acid, linolenic acid, arachidic acid, arachidonic acid, behenic acid, and lignoceric acid. When the glycerophospholipids and sphingophospholipids have two or more fatty acid residues, all the fatty acid residues may be the same group or different groups.

The sterols are not particularly limited but, examples thereof include animal-derived sterols such as cholesterol, cholesterol hemisuccinate, lanosterol, dihydrolanosterol, desmosterol, and dihydrocholesterol; plant-derived sterols (phytosterols) such as stigmasterol, sitosterol, campesterol, and brassicasterol; and microorganism-derived sterols such as timosterol and ergosterol.

The glycolipids are not particularly limited but, examples thereof include glycoglycerolipids such as sulfoxyribosyl glyceride, diglycosyl diglyceride, digalactosyl diglyceride, galactosyl diglyceride, and glycosyl diglyceride; and glycosphingolipids such as galactosylceramide, lactosylceramide, and gangliosides.

The saturated or unsaturated fatty acids are not particularly limited but, examples thereof include saturated or unsaturated fatty acids having about 12 to about 20 carbon atoms such as palmitic acid, oleic acid, stearic acid, arachidonic acid, and myristic acid.

In a certain embodiment, the lipid nanoparticle of the present invention may further comprise a polyalkylene glycol-modified lipid as a constituent lipid. Since polyalkylene glycol is a hydrophilic polymer, the surface of a lipid nanoparticle can be modified with polyalkylene glycol by constructing the lipid nanoparticle using the polyalkylene glycol-modified lipid as a constituent lipid. Surface modification with polyalkylene glycol may enhance the stability of the lipid nanoparticle such as the blood retention property. The polyalkylene glycol is not particularly limited but, as the polyalkylene glycol, for example, polyethylene glycol, polypropylene glycol, polytetramethylene glycol, polyhexamethylene glycol, or the like can be used. The molecular weight of the polyalkylene glycol is not particularly limited, but may be, for example, about 300 to about 10,000, preferably about 500 to about 10,000, and more preferably about 1,000 to about 5,000.

The polyalkylene glycol-modified lipid is not particularly limited but, as the polyalkylene glycol-modified lipid, for example, stearylated polyethylene glycol (e.g., stearic acid PEG45 (STR-PEG45)) can be used. In addition, polyethylene glycol derivatives such as N-[carbonyl-methoxypolyethylene glycol-2000]-1,2-dipalmitoyl-sn-glycero-3-phosphoethanolamine, N-[carbonyl-methoxypolyethylene glycol-5000]-1,2-dipalmitoyl-sn-glycero-3-phosphoethanolamine, N-[carbonyl-methoxypolyethylene glycol-750]-1,2-distearoyl-sn-glycero-3-phosphoethanolamine, N-[carbonyl-methoxypolyethylene glycol-2000]-1,2-distearoyl-sn-glycero-3-phosphoethanolamine, N-[carbonyl-methoxypolyethylene glycol-5000]-1,2-distearoyl-sn-glycero-3-phosphoethanolamine, and 1,2-dimyristoyl-rac-glycero-3-methoxypolyethylene glycol-2000 (PEG-DMG) can also be used, but polyalkylene glycolated lipids are not limited thereto.

The proportion of the polyalkylene glycol-modified lipid is not particularly limited as long as it does not impair the effect of suppressing the inhibition of phase transition of phosphatidylcholine, but may be, for example, about 0.5 to about 3 mol% relative to the total lipid amount in the lipid nanoparticle of the present invention.

The lipid nanoparticle of the present invention can be subjected to appropriate surface modification or the like as necessary. As an example, the blood retention property of the lipid nanoparticle of the present invention can be enhanced by modifying the surface thereof with a hydrophilic polymer or the like. Surface modification may be performed by using a lipid modified with these modifying groups as a constituent lipid of the lipid nanoparticle. Lipid derivatives for enhancing the blood retention property of the lipid nanoparticle of the present invention are not particularly limited, but examples thereof include glycophorin, ganglioside GM1, phosphatidylinositol, ganglioside GM3, glucuronic acid derivative, glutamic acid derivative, and polyglycerin phospholipid derivative. The hydrophilic polymer for enhancing the blood retention property also is not particularly limited, but examples thereof include dextran, pullulan, Ficoll, polyvinyl alcohol, styrene-maleic anhydride alternating copolymer, divinyl ether-maleic anhydride alternating copolymer, amylose, amylopectin, chitosan, mannan, cyclodextrin, pectin, and carrageenan in addition to the aforementioned polyalkylene glycol.

Further, in order to promote nuclear translocation of the lipid nanoparticle of the present invention, although not particularly limited, for example, the surface of the lipid nanoparticle may be modified with an oligosaccharide compound having three or more saccharides. The oligosaccharide compound is not particularly limited, but may be, for example, an oligosaccharide compound in which about 3 to about 10 sugar units are bonded, and preferably an oligosaccharide compound in which about 3 to about 6 sugar units are bonded. Specifically, the oligosaccharide compound may be an oligosaccharide compound which is a trimer to hexamer of glucose, and preferably an oligosaccharide compound which is a trimer or tetramer of glucose. More specifically, the oligosaccharide compound may be isomaltotriose, isopanose, maltotriose, maltotetraose, maltopentaose, maltohexaose, or the like, and preferably maltotriose, maltotetraose, maltopentaose, or maltohexaose in which glucoses are bonded through an α1-4 linkage.

The lipid nanoparticle of the present invention can be imparted with, for example, any one or two or more functions selected from the group consisting of a temperature change-sensitive function, a membrane permeation function, a gene expression function, a pH-sensitive function, and the like. By appropriately adding these functions, it is possible to improve the retention property of the lipid nanoparticle in blood, efficiently release the lipid nanoparticle from the endosome after endocytosis in the target cell, and more efficiently express the encapsulated nucleic acid in the target cell.

The lipid nanoparticle of the present invention may further comprise any optional components that can be commonly used in the art or other components useful for the efficient delivery of the contents of the lipid nanoparticle into cells, provided that the objectives of the present invention are not compromised. The optional components are not particularly limited, but examples thereof include antioxidants such as tocopherol, propyl gallate, ascorbyl palmitate, and butylated hydroxytoluene, charged substances, and membrane polypeptides. Among the charged substances, examples of those for imparting a positive charge include saturated or unsaturated aliphatic amines such as stearylamine and oleylamine, and examples of those for imparting a negative charge include dicetyl phosphate, cholesteryl hemisuccinate, phosphatidylserine, phosphatidylinositol, and phosphatidic acid. Examples of membrane polypeptides include peripheral membrane polypeptides and integral membrane polypeptides. The blending amount of these substances is not particularly limited and can be appropriately selected depending on the purposes.

The size of the lipid nanoparticle of the present invention is not particularly limited, but the lipid nanoparticle may have, for example, an average particle size of about 400 nm or less, preferably about 300 nm or less, more preferably about 200 nm or less, and further preferably about 150 nm or less. The lipid nanoparticle of the present invention may also have an average particle size of about 10 nm or more, preferably about 30 nm or more, more preferably about 40 nm or more, and further preferably about 50 nm or more. When the average particle size of the lipid nanoparticle falls within such ranges, it is considered that delivery efficiency to target cells within the body tends to be high, and good activity is also likely to be exhibited. The average particle size of the lipid nanoparticle means a number average particle size measured by dynamic light scattering (DLS). Measurement by dynamic light scattering can be carried out by a conventional method using a commercially available DLS apparatus or the like.

The polydispersity index (PDI) of the lipid nanoparticle of the present invention is not particularly limited, but may be, for example, about 0.01 to about 0.7, preferably about 0.01 to about 0.6, more preferably about 0.01 to about 0.3. The zeta potential of the lipid nanoparticle of the present invention is not particularly limited, but may be, for example, in the range of -50 mV to 5 mV, preferably in the range of -10 mV to 5 mV.

The form of the lipid nanoparticle of the present invention is not particularly limited, but may be, for example, a unilamellar liposome, a multilamellar liposome, a spherical micelle, or an amorphous layered structure, as a form dispersed in an aqueous solvent. In a certain embodiment, the lipid nanoparticle of the present invention may be a unilamellar liposome or a multilamellar liposome.

The lipid nanoparticle of the present invention may encapsulate a substance to be delivered into target cells (delivered substance) within the particle interior covered by a lipid membrane. The delivered substance is not particularly limited as long as it can be encapsulated in the lipid nanoparticle, but may comprise, for example, a nucleic acid, a saccharide, a peptide, a low molecular weight compound, and/or a metal compound.

The nucleic acid as the delivered substance is not particularly limited, but may comprise DNA and/or RNA, or an analog or derivative thereof (e.g., peptide nucleic acid (PNA) or phosphorothioate DNA). The nucleic acid may be a single-stranded nucleic acid or a double-stranded nucleic acid and may be linear or circular.

The nucleic acid as the delivered substance may comprise a foreign gene to be expressed in a target cell. In other words, the nucleic acid may be a nucleic acid that functions to express the foreign gene in a cell by being taken up into the cell. The foreign gene may be a gene originally contained in the genomic DNA of the target cell or a gene not contained in the genomic DNA. Examples of such nucleic acids include gene expression vectors containing a nucleic acid having a base sequence encoding a gene to be expressed. The gene expression vector may exist as an extrachromosomal gene in a transfected cell or may be integrated into genomic DNA by homologous recombination.

As the gene expression vector, a vector generally used in gene therapy or the like can be used without any particular limitation, and the gene expression vector may be, for example, a nucleic acid vector such as a plasmid vector. When encapsulated in the lipid nanoparticle of the present invention, the plasmid vector may be in a circular form or in a state of being linearly cleaved in advance. The gene expression vector can be designed by a conventional method with generally used molecular biological tools based on the base sequence information of the target gene to be expressed, and can be produced by various known methods.

The nucleic acid as the delivered substance may be a functional nucleic acid that controls expression of a target gene present in a target cell. The functional nucleic acid is not particularly limited, but examples thereof include antisense oligonucleotides, antisense DNA, antisense RNA, siRNA, microRNA, mRNA, and gRNA. The functional nucleic acid may also be plasmid DNA (pDNA) serving as an expression vector such as an siRNA expression vector that expresses siRNA in a cell. The expression vector can be prepared from a commercial product and can be modified appropriately. For example, the siRNA expression vector can be prepared from a commercially available siRNA expression vector, or can be appropriately modified. Encapsulating the nucleic acid in the lipid nanoparticle of the present invention enables its efficient delivery to tissues within the body, making it particularly useful for delivering mRNA or pDNA.

The lipid nanoparticle of the present invention can be produced using any commonly employed method in the art. It can be produced as follows: for example, all lipid components are dissolved in an organic solvent such as chloroform, and a lipid membrane is formed by drying under reduced pressure using an evaporator or spray drying using a spray dryer, and then an aqueous solvent containing a component to be encapsulated in the lipid nanoparticle (e.g., a nucleic acid) is added to the dried lipid membrane, and further emulsified by an emulsifier such as a homogenizer, an ultrasonic emulsifier, or a high-pressure jet emulsifier. The lipid nanoparticle of the present invention can also be produced using a method well known as a method for producing liposomes, such as a reverse phase evaporation method. When it is desired to control the size of the lipid nanoparticle, extrusion through a membrane filter having a uniform pore size or the like under high pressure may be performed.

The aqueous solvent (dispersion medium) is not particularly limited, but may be, for example, a buffer solution such as phosphate buffer, citrate buffer, and phosphate buffered saline, physiological saline, or cell culture medium. These aqueous solvents (dispersion media) can stably disperse the lipid nanoparticles, but further, sugars, for example, monosaccharides such as glucose, galactose, mannose, fructose, inositol, ribose, and xylose, disaccharides such as lactose, sucrose, cellobiose, trehalose, and maltose, trisaccharides such as raffinose and melezitose, polysaccharides such as dextrin and cyclodextrin, and sugar alcohols such as erythritol, xylitol, sorbitol, mannitol, and maltitol (or aqueous solutions of the sugars), and polyhydric alcohols such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol (or aqueous solutions thereof) may be added. In order to stably store the lipid nanoparticle dispersed in the aqueous solvent for a long period of time, it is desirable to exclude electrolytes in the aqueous solvent as much as possible in view of physical stability such as aggregation suppression. Also, in view of chemical stability of lipids, a pH of the aqueous solvent may be set to near neutral from weakly acidic (a pH of about 3.0 to about 8.0) and dissolved oxygen may be removed by nitrogen bubbling or the like.

The lipid nanoparticle of the present invention can also be produced by an alcohol dilution method using a flow path. The method is a method for producing lipid nanoparticles by introducing, from separate flow paths, a solution in which lipid components are dissolved in an alcohol solvent and a solution in which a water-soluble component to be included in the lipid nanoparticle is dissolved in an aqueous solvent and merging them. By using a microchannel with a built-in three-dimensional micromixer capable of achieving instantaneous mixing of two liquids, lipid nanoparticles having a diameter of about 30 nm can be reproducibly produced (Non-patent Literature 6). As the flow path used for production, a simple two-dimensional flow path structure in which baffles of a certain width are arranged alternately from both sides in a micro-sized flow path through which a raw material solution flows, as described in Patent Literature 3 and Non-patent Literature 7, may be used, since it can form a nano-sized lipid particle formation system with high particle size controllability. As the aqueous solvent used in the alcohol dilution method, the solvents described in the above paragraph can be used.

When the resulting aqueous dispersion of the lipid nanoparticle is freeze-dried or spray-dried, as an example, a use of sugars, for example, monosaccharides such as glucose, galactose, mannose, fructose, inositol, ribose, and xylose, disaccharides such as lactose, sucrose, cellobiose, trehalose, and maltose, trisaccharides such as raffinose and melezitose, polysaccharides such as dextrin and cyclodextrin, and sugar alcohols such as erythritol, xylitol, sorbitol, mannitol, and maltitol (or aqueous solutions of the sugars) may enhance stability. Further, when the aqueous dispersion is frozen, for example, a use of the above-mentioned sugars or polyhydric alcohols such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol (or aqueous solutions thereof) may enhance stability.

The lipid nanoparticle of the present invention may function as a gene expression carrier to a target tissue. By encapsulating a foreign gene of interest to be expressed in a target cell in the lipid nanoparticle and administering the lipid nanoparticle to a subject animal, the foreign gene is expressed in the target tissue of the subject animal. Therefore, the lipid nanoparticle of the present invention is useful as an ingredient for gene therapy or an experimental reagent.

In another embodiment, the present invention relates to a composition comprising a lipid nanoparticle comprising the compound represented by the formula (I). The compound represented by the formula (I) and the lipid nanoparticle are as described herein as embodiments of the present invention, respectively. In a certain embodiment, the composition of the present invention may be a composition for expressing a foreign gene and/or for use in gene therapy.

A subject animal to which the composition of the present invention is applied is not particularly limited and may be a human or an animal other than a human. Examples of non-human animals include mammals such as cattle, pigs, horses, sheep, goats, monkeys, dogs, cats, rabbits, mice, rats, hamsters, and guinea pigs, and birds such as chickens, quails, and ducks. The administration route for administering the composition of the present invention to the subject animal is not particularly limited, but may be, for example, parenteral administration such as intravenous administration, enteral administration, intramuscular administration, subcutaneous administration, transdermal administration, nasal administration, and pulmonary administration.

In one embodiment, the lipid nanoparticle of the present invention or the composition of the present invention is for use as a medicament, for example, a medicament for gene therapy. Furthermore, in one embodiment, a use of the lipid nanoparticle of the present invention or the composition of the present invention in the manufacture of a medicament, for example, a medicament for gene therapy, is provided.

In one embodiment, a method for expressing a foreign gene in a target cell of a subject animal, comprising administering to the subject animal the lipid nanoparticle of the present invention encapsulating the foreign gene to be expressed in the target cell or the composition comprising the lipid nanoparticle, is provided. In another embodiment, a method for controlling expression of a target gene, comprising administering to a subject animal the lipid nanoparticle of the present invention encapsulating a nucleic acid capable of controlling expression of the target gene or the composition comprising the lipid nanoparticle, is provided.

In one embodiment, the lipid nanoparticle of the present invention or the composition of the present invention can be used in a method for treating or preventing a disease associated with an abnormality in a gene. Specifically, a method for treating or preventing a disease associated with an abnormality in a gene is provided, the method comprising administering to a subject in need thereof the lipid nanoparticle of the present invention comprising a nucleic acid or the composition comprising the lipid nanoparticle, wherein the nucleic acid suppresses or substitutes for the function of the gene.

The composition of the present invention may further comprise any optional components that can be commonly used in the art and may further comprise additional active ingredients, provided that the objectives of the present invention are not compromised.

The following examples specifically describe the present invention, but the scope of the present invention is not limited to these examples.

### Examples

### [Synthesis Examples]

### (1) Methods and materials

¹H and ¹³C NMR spectra were obtained using a JEOL ECA500 instrument with tetramethylsilane as the internal standard (0 ppm). The following abbreviations were used: s = singlet; d = doublet; t = triplet; and m = multiplet. The chemical shifts of ¹H NMR spectra are reported in ppm on sigma scale downfield from tetramethylsilane. All reactions were monitored by thin-layer chromatography (TLC) on pre-coated TLC plates (Millipore), and TLC visualization was performed by UV light (254 nm), phosphomolybdic acid stain or p-anisaldehyde, iodine, ninhydrin, or Dragendorffs reagent stain. The reaction products were purified by Biotage^{(R)} Selekt system equipped with evaporative light scattering detector. All simple chemicals were purchased from commercial sources and were used without further purification. The mass spectrometry was performed using an expression CMS equipped with plate express (Advion Interchim Scientific).

### (2) Synthesis of (R)-2,3-bis(oleoyloxy)propyl (2-((3-butoxy-3-oxopropyl)ammonio)ethyl) phosphate (DOPE-C4)

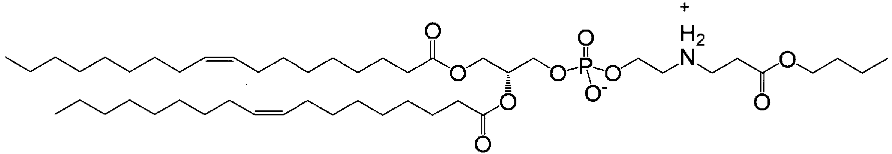

DOPE (224.4 mg, 0.30 mmol), butyl acrylate (42.3 mg, 0.33 mmol), and diisopropylethylamine (DIPEA) (102 µL, 0.60 mmol) were dissolved in chloroform (1.5 mL) and were stirred at 50°C for 2 days under Ar atmosphere. Solvent was removed in vacuo, and then the residue was loaded on normal-phase column (Sfär Silica HC D, Biotage), and purified by flash chromatography with a gradient mobile phase of dichloromethane (DCM) and methanol (MeOH). This gave 137.8 mg (52.6%) of DOPE-C4 as a pale yellow solid.
¹H NMR (500 MHz, CDCl₃) δ: 0.88 (t, 6H), 0.92 (t, 3H), 1.22-1.40 (m, 42H), 1.60 (m, 6H), 2.00 (m, 8H), 2.28 (m, 4H), 2.94 (t, 2H), 3.14-3.26 (m, 4H), 4.00-4.19 (m, 5H), 4.24 (br, 2H), 4.37 (m, 1H), 5.22 (m, 1H), 5.33 (m, 4H).

### (3) Synthesis of (R)-2,3-bis(oleoyloxy)propyl (2-((3-(hexyloxy)-3-oxopropyl)ammonio)ethyl) phosphate (DOPE-C6)

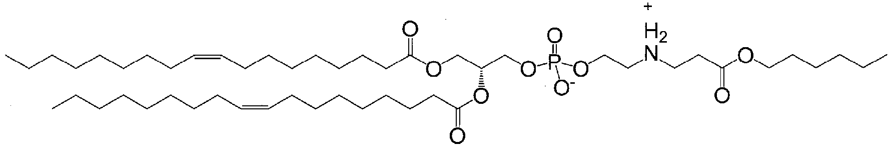

DOPE (224.4 mg, 0.30 mmol), hexyl acrylate (51.6 mg, 0.33 mmol), and DIPEA (102 µL, 0.60 mmol) were dissolved in chloroform (1.5 mL) and were stirred at 50°C for 2 days under Ar atmosphere. Solvent was removed in vacuo, and then the residue was loaded on normal-phase column (Sfär Silica HC D, Biotage), and purified by flash chromatography with a gradient mobile phase of dichloromethane (DCM) and methanol (MeOH). This gave 147.4 mg (54.6%) of DOPE-C6 as a pale yellow solid. ¹H NMR (500 MHz, CDCl₃) δ: 0.88 (t, 9H), 1.22-1.37 (m, 46H), 1.60 (m, 6H), 2.00 (m, 8H), 2.28 (m, 4H), 2.98 (t, 2H), 3.14-3.28 (m, 4H), 3.96-4.17 (m, 5H), 4.26 (br, 2H), 4.36 (m, 1H), 5.21 (m, 1H), 5.33 (m, 4H).

### (4) Synthesis of (R)-2,3-bis(oleoyloxy)propyl (2-((3-(octyloxy)-3-oxopropyl)ammonio)ethyl) phosphate (DOPE-C8)

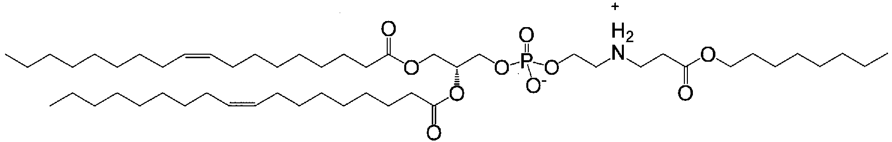

DOPE (224.4 mg, 0.30 mmol), n-octyl acrylate (60.8 mg, 0.33 mmol), and triethylamine (TEA) (55.8 µL, 0.40 mmol) were dissolved in chloroform (1.5 mL) and were stirred at 50°C for 2 days under Ar atmosphere. Solvent was removed in vacuo, and then the residue was loaded on normal-phase column (Sfär Silica HC D, Biotage), and purified by flash chromatography with a gradient mobile phase of dichloromethane (DCM) and methanol (MeOH). This gave 132.3 mg (47.6%) of DOPE-C8 as a white solid.
¹H NMR (500 MHz, CD₃OD) δ: 0.88 (t, 9H), 1.22-1.40 (m, 50H), 1.62 (m, 6H), 2.02 (m, 8H), 2.32 (m, 4H), 2.80 (t, 2H), 3.33 (t, 4H), 3.99 (t, 2H), 4.05-4.19 (m, 5H), 4.42 (m, 1H), 5.22 (m, 1H), 5.33 (m, 4H).

### (5) Synthesis of (R)-2,3-bis(oleoyloxy)propyl (2-((3-(decyloxy)-3-oxopropyl)ammonio)ethyl) phosphate (DOPE-C10)

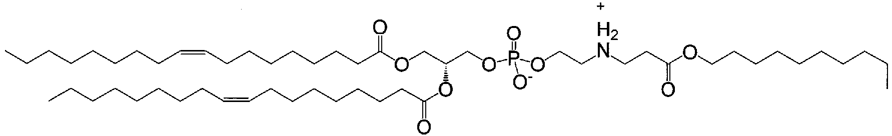

1-Decanol (158 mg, 1.0 mmol) and TEA (209 µL, 1.20 mmol) were dissolved in anhydrous DCM and then were added with acryloyl chloride (111 mg, 1.20 mmol) dropwise in ice bath. The reaction mixture was stirred at ambient temperature for 2 hours. Solvent was removed in vacuo, and then the residue was dissolved in ethyl acetate (AcOEt) and was washed with water and brine. The organic phase was dried over with anhydrous Na₂SO₄. After removal of solvent in vacuo, the crude product was dissolved in a mixture of hexane and AcOEt (9:1) and then was passed through Wakogel C-100. The filtrate was concentrated in vacuo, and the crude product (decyl acrylate) was used for the next step without further purification.

DOPE (299.0 mg, 0.40 mmol), decyl acrylate (93.4 mg, 0.44 mmol), and DIPEA (136 µL, 0.80 mmol) were dissolved in chloroform (2.0 mL) and were stirred at 60°C over night under Ar atmosphere. Solvent was removed in vacuo, and then the residue was loaded on normal-phase column (Sfär Silica HC D, Biotage), and purified by flash chromatography with a gradient mobile phase of dichloromethane (DCM) and methanol (MeOH). This gave 128.8 mg (33.8% from DOPE) of DOPE-C10 as a pale yellow solid.
¹H NMR (500 MHz, CDCl₃) δ: 0.88 (t, 9H), 1.20-1.40 (m, 70H), 1.60 (m, 6H), 2.00 (m, 8H), 2.28 (m, 4H), 2.88 (t, 2H), 3.19 (t, 4H), 3.97-4.26 (m, 7H), 4.36 (m, 1H), 5.22 (m, 1H), 5.33 (m, 4H).

### (6) Synthesis of (R)-2,3-bis(oleoyloxy)propyl (2-((3-(dodecyloxy)-3-oxopropyl)ammonio)ethyl) phosphate (DOPE-C12)

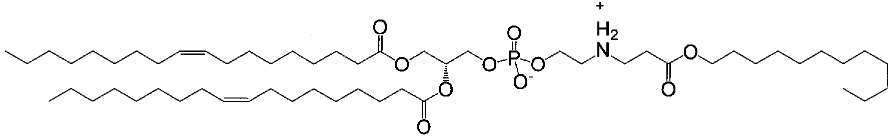

DOPE (224.4 mg, 0.30 mmol), dodecyl acrylate (79.3 mg, 0.33 mmol), and TEA (55.8 µL, 0.40 mmol) were dissolved in chloroform (1.5 mL) and were stirred at 50°C for 2 days under Ar atmosphere. Solvent was removed in vacuo, and then the residue was loaded on normal-phase column (Sfär Silica HC D, Biotage), and purified by flash chromatography with a gradient mobile phase of dichloromethane (DCM) and methanol (MeOH). This gave 159.0 mg (54.0%) of DOPE-C12 as a pale yellow solid. ¹H NMR (500 MHz, CDCl₃) δ: 0.88 (t, 9H), 1.20-1.40 (m, 58H), 1.60 (m, 6H), 2.00 (m, 8H), 2.28 (m, 4H), 2.88 (t, 2H), 3.19 (t, 4H), 3.97-4.26 (m, 7H), 4.39 (m, 1H), 5.22 (m, 1H), 5.33 (m, 4H).

### (7) Synthesis of (R)-2,3-bis(oleoyloxy)propyl (2-((3-(octadecyloxy)-3-oxopropyl)ammonio)ethyl) phosphate (DOPE-C18)

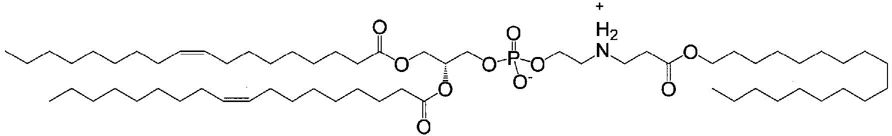

DOPE (224.4 mg, 0.30 mmol), stearyl acrylate (107.1 mg, 0.33 mmol), and TEA (55.8 µL, 0.40 mmol) were dissolved in chloroform (1.5 mL) and were stirred at 50°C for 2 days under Ar atmosphere. Solvent was removed in vacuo, and then the residue was loaded on normal-phase column (Sfär Silica HC D, Biotage), and purified by flash chromatography with a gradient mobile phase of dichloromethane (DCM) and methanol (MeOH). This gave 165.0 mg (51.3%) of DOPE-C18 as a white solid.
¹H NMR (500 MHz, CDCl₃) δ: 0.88 (t, 9H), 1.20-1.40 (m, 70H), 1.60 (m, 6H), 2.00 (m, 8H), 2.28 (m, 4H), 2.88 (t, 2H), 3.19 (t, 4H), 3.97-4.26 (m, 7H), 4.36 (m, 1H), 5.22 (m, 1H), 5.33 (m, 4H).

### (8) Synthesis of (R)-2,3-bis(oleoyloxy)propyl (2-((3-(((Z)-octadeca-9-ene-1-yl)oxy)-3-oxopropyl)ammonio)ethyl) phosphate (DOPE-C18:1)

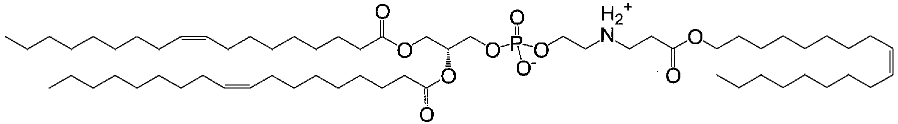

Oleyl alcohol (269 mg, 1.0 mmol) and TEA (209 µL, 1.20 mmol) were dissolved in anhydrous DCM and then were added with acryloyl chloride (90.5 mg, 1.0 mmol) dropwise in ice bath. The reaction mixture was stirred at ambient temperature for 1 hour. Solvent was removed in vacuo, and then the residue was suspended in hexane and was filtered. The filtrate loaded on normal-phase column (Sfär Silica HC D, Biotage), and purified by flash chromatography with a gradient mobile phase of hexane and AcOEt. This gave oleyl acrylate as a colorless oil.

DOPE (299.0 mg, 0.40 mmol), oleyl acrylate (142.0 mg, 0.44 mmol), and DIPEA (136 µL, 0.80 mmol) were dissolved in chloroform (2.0 mL) and were stirred at 60°C over night under Ar atmosphere. Solvent was removed in vacuo, and then the residue was loaded on normal-phase column (Sfär Silica HC D, Biotage), and purified by flash chromatography with a gradient mobile phase of dichloromethane (DCM) and methanol (MeOH). This gave 152.4 mg (35.8% from DOPE) of DOPE-C18:1 as an yellow solid.
¹H NMR (500 MHz, CDCl₃) δ: 0.88 (t, 9H), 1.20-1.40 (m, 62H), 1.60 (m, 6H), 2.00 (m, 12H), 2.29 (m, 4H), 2.89 (br, 2H), 3.19 (br, 4H), 3.97-4.26 (m, 7H), 4.38 (m, 1H), 5.22 (m, 1H), 5.33 (m, 6H).

### (9) Synthesis of (R)-2,3-bis(oleoyloxy)propyl (2-((3-((2-methylpentyl)oxy)-3-oxopropyl)ammonio)ethyl) phosphate (DOPE-C5_β-1)

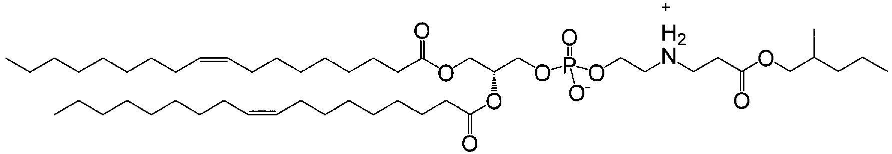

2-Methyl-1-pentanol (102.2 mg, 1.0 mmol) and TEA (209 µL, 1.50 mmol) were dissolved in anhydrous DCM (4 mL) and then were added with acryloyl chloride (80.8 µL, 1.0 mmol) dropwise in ice bath. The reaction mixture was stirred at ambient temperature for 2 hours. Solvent was removed in vacuo, and then the residue was suspended in hexane/AcOEt (9:1) and was passed through silica pad. The filtrate was concentrated in vacuo. The crude product (2-methylpentyl acrylate) was used for the next reaction without further purification.

DOPE (598.5 mg, 0.80 mmol), the crude product (2-methylpentyl acrylate) (107.1 mg), and TEA (209 µL, 1.50 mol) were dissolved in chloroform (4 mL) and were stirred at 60°C for 2 days under Ar atmosphere. The reaction mixture was loaded on normal-phase column (Sfär Silica HC D, Biotage), and purified by flash chromatography with a gradient mobile phase of dichloromethane (DCM) and methanol (MeOH). This gave 284.3 mg (39.5%) of DOPE-C5_β-1 as a yellow viscous oil.
¹H NMR (500 MHz, CD₃OD) δ: 0.88 (t, 12H), 1.20-1.43 (m, 44H), 1.60 (m, 4H), 1.80 (m, 1H), 2.00 (m, 8H), 2.31 (m, 4H), 2.80 (t, 2H), 3.19 (t, 2H), 3.33 (t, 2H), 3.90-4.20 (m, 7H), 4.42 (m, 1H), 5.22 (m, 1H), 5.33 (m, 4H)
MS (ESI): m/z calculated for C₅₀H₉₃NO₁₀P (M-H)⁻, 898.7; found, 898.7.

### (10) Synthesis of (R)-2,3-bis(oleoyloxy)propyl (2-((3-((2-ethylhexyl)oxy)-3-oxopropyl)ammonio)ethyl) phosphate (DOPE-C6_β-2)

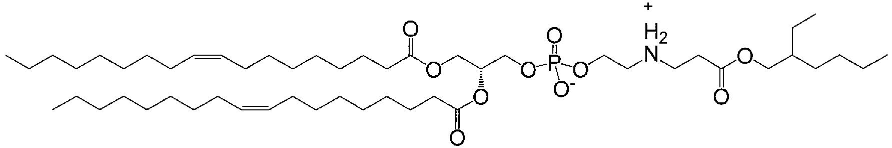

2-Ethyl-1-hexanol (130.2 mg, 1.0 mmol) and TEA (209 µL, 1.50 mmol) were dissolved in anhydrous DCM (4 mL) and then were added with acryloyl chloride (80.8 µL, 1.0 mmol) dropwise in ice bath. The reaction mixture was stirred at ambient temperature for 2 hours. Solvent was removed in vacuo, and then the residue was suspended in hexane/AcOEt (9:1) and was passed through silica pad. The filtrate was concentrated in vacuo. The crude product (2-ethylhexyl acrylate) was used for the next reaction without further purification.

DOPE (598.5 mg, 0.80 mmol), the crude product (2-ethylhexyl acrylate) (158.0 mg), and TEA (209 µL, 1.50 mol) were dissolved in chloroform (4 mL) and were stirred at 60°C for 2 days under Ar atmosphere. The reaction mixture was loaded on normal-phase column (Sfär Silica HC D, Biotage), and purified by flash chromatography with a gradient mobile phase of dichloromethane (DCM) and methanol (MeOH). This gave 524.3 mg (70.6%) of DOPE-C6_β-2 as a yellow viscous oil.
¹H NMR (500 MHz, CD₃OD) δ: 0.88 (t, 12H), 1.21-1.43 (m, 48H), 1.60 (m, 5H), 2.01 (m, 8H), 2.31 (m, 4H), 2.80 (t, 2H), 3.19 (t, 2H), 3.33 (t, 2H), 3.96-4.18 (m, 7H), 4.42 (m, 1H), 5.22 (m, 1H), 5.33 (m, 4H).
MS (ESI): m/z calculated for C₅₂H₉₇NO₁₀P (M-H)⁻, 926.7; found, 926.7.

### (11) Synthesis of (R)-2,3-bis(oleoyloxy)propyl (2-((3-oxo-3-((2-propylheptyl)oxy)propyl)ammonio)ethyl) phosphate (DOPE-C7_β-3)

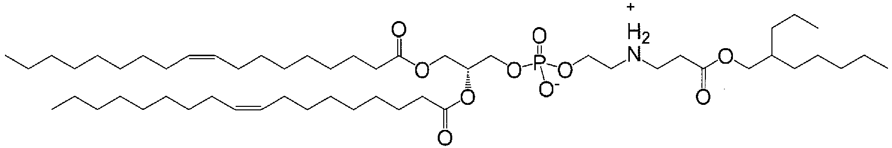

2-propyl-1-heptan-1-ol (158.3 mg, 1.0 mmol) and TEA (209 µL, 1.50 mmol) were dissolved in anhydrous DCM (4 mL) and then were added with acryloyl chloride (80.8 µL, 1.0 mmol) dropwise in ice bath. The reaction mixture was stirred at ambient temperature for 2 hours. Solvent was removed in vacuo, and then the residue was suspended in hexane/AcOEt (9:1) and was passed through silica pad. The filtrate was concentrated in vacuo. The crude product (2-propylheptyl acrylate) was used for the next reaction without further purification.

DOPE (598.5 mg, 0.80 mmol), the crude product (2-propylheptyl acrylate) (152.3 mg), and TEA (209 µL, 1.50 mol) were dissolved in chloroform (4 mL) and were stirred at 60°C for 2 days under Ar atmosphere. The reaction mixture was loaded on normal-phase column (Sfär Silica HC D, Biotage), and purified by flash chromatography with a gradient mobile phase of dichloromethane (DCM) and methanol (MeOH). This gave 307 mg (40.1%) of DOPE-C7_β-3 as a pale yellow viscous oil.
¹H NMR (500 MHz, CD₃OD) δ: 0.88 (t, 12H), 1.21-1.40 (m, 52H), 1.60 (m, 4H), 1.66 (m, 1H), 2.01 (m, 8H), 2.31 (m, 4H), 2.80 (t, 2H), 3.33 (t, 4H), 3.96-4.18 (m, 7H), 4.42 (m, 1H), 5.22 (m, 1H), 5.33 (m, 4H).
MS (ESI): m/z calculated for C₅₄H₁₀₁NO₁₀P (M-H)⁻, 954.7; found, 954.7.

### (12) Synthesis of (R)-2,3-bis(oleoyloxy)propyl (2-((3-((2-butyloctyl)oxy)-3-oxopropyl)ammonio)ethyl) phosphate (DOPE-C8_β-4)

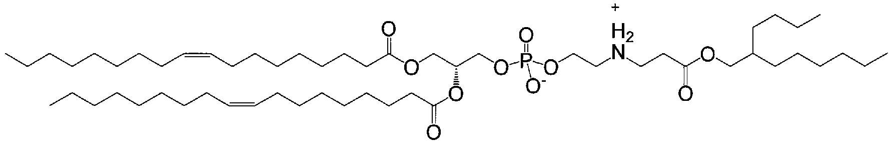

2-butyl-1-n-octanol (186.3 mg, 1.0 mmol) and TEA (209 µL, 1.50 mmol) were dissolved in anhydrous DCM (4 mL) and then were added with acryloyl chloride (80.8 µL, 1.0 mmol) dropwise in ice bath. The reaction mixture was stirred at ambient temperature for 2 hours. Solvent was removed in vacuo, and then the residue was suspended in hexane/AcOEt (9:1) and was passed through silica pad. The filtrate was concentrated in vacuo. The crude product (2-butyloctyl acrylate) was used for the next reaction without further purification.

DOPE (598.5 mg, 0.80 mmol), the crude product (2-butyloctyl acrylate) (190.1 mg), and TEA (209 µL, 1.50 mol) were dissolved in chloroform (4 mL) and were stirred at 60°C for 2 days under Ar atmosphere. The reaction mixture was loaded on normal-phase column (Sfär Silica HC D, Biotage), and purified by flash chromatography with a gradient mobile phase of dichloromethane (DCM) and methanol (MeOH). This gave 303 mg (38.5%) of DOPE-C8_P-4 as a pale yellow viscous oil.
¹H NMR (500 MHz, CD₃OD) δ: 0.88 (t, 12H), 1.21-1.39 (m, 56H), 1.60 (m, 4H), 1.65 (m, 1H), 2.01 (m, 8H), 2.31 (m, 4H), 2.80 (t, 2H), 3.33 (t, 4H), 3.96-4.18 (m, 7H), 4.42 (m, 1H), 5.22 (m, 1H), 5.33 (m, 4H).
MS (ESI): m/z calculated for C₅₆H₁₀₅NO₁₀P (M-H)⁻, 982.7; found, 982.7.

### (13) Synthesis of (R)-2,3-bis(oleoyloxy)propyl (2-((3-((3,7-dimethyloct-6-en-1-yl)oxy)-3-oxopropyl)ammonio)ethyl) phosphate (DOPE-Cit)

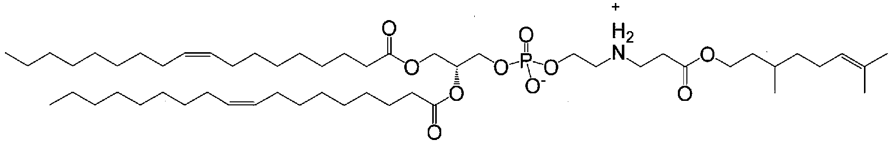

β-citronellol (156.3 mg, 1.0 mmol) and TEA (209 µL, 1.50 mmol) were dissolved in anhydrous DCM (4 mL) and then were added with acryloyl chloride (80.8 µL, 1.0 mmol) dropwise in ice bath. The reaction mixture was stirred at ambient temperature for 2 hours. Solvent was removed in vacuo, and then the residue was suspended in hexane/AcOEt (9:1) and was passed through silica pad. The filtrate was concentrated in vacuo. The crude product (3,7-dimethyloct-6-en-1-yl acrylate) was used for the next reaction without further purification.

DOPE (598.5 mg, 0.80 mmol), the crude product (3,7-dimethyloct-6-en-1-yl acrylate) (150.1 mg), and TEA (209 µL, 1.50 mol) were dissolved in chloroform (4 mL) and were stirred at 60°C for 2 days under Ar atmosphere. The reaction mixture was loaded on normal-phase column (Sfär Silica HC D, Biotage), and purified by flash chromatography with a gradient mobile phase of dichloromethane (DCM) and methanol (MeOH). This gave 98.6 mg (12.9%) of DOPE-Cit as a pale yellow solid.
¹H NMR (500 MHz, CD₃OD) δ: 0.88 (t, 9H), 122-1.39 (m, 43H), 1.60 (m, 7H), 1.65 (s, 3H), 2.01 (m, 10H), 2.31 (m, 4H), 2.80 (t, 2H), 3.19 (t, 2H), 3.33 (t, 4H), 3.96-4.20 (m, 7H), 4.42 (m, 1H), 5.09 (t, 1H), 5.22 (m, 1H), 5.33 (m, 4H).
MS (ESI): m/z calculated for C₅₄H₉₉NO₁₀P (M-H)⁻, 952.7; found, 952.7.

### (14) Synthesis of (R)-2,3-bis(oleoyloxy)propyl (2-((3-oxo-3-(pentan-3-yloxy)propyl)ammonio)ethyl) phosphate (DOPE-C3_α-2)

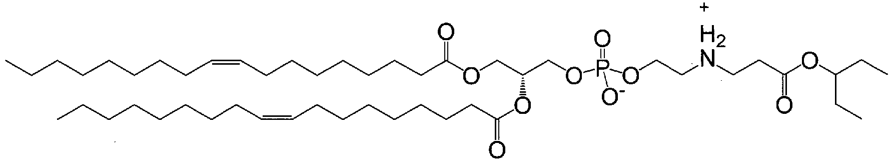

3-Pentanol (88.2 mg, 1.0 mmol) and TEA (209 µL, 1.50 mmol) were dissolved in anhydrous DCM (4 mL) and then were added with acryloyl chloride (80.8 µL, 1.0 mmol) dropwise in ice bath. The reaction mixture was stirred at ambient temperature for 2 hours. Solvent was removed in vacuo, and then the residue was suspended in hexane/AcOEt (9:1) and was passed through silica pad. The filtrate was concentrated in vacuo. The crude product (pentan-3-yl acrylate) was used for the next reaction without further purification.

DOPE (598.5 mg, 0.80 mmol), the crude product (pentan-3-yl acrylate) (135 mg), and TEA (209 µL, 1.50 mol) were dissolved in chloroform (4 mL) and were stirred at 60°C for 2 days under Ar atmosphere. The reaction mixture was loaded on normal-phase column (Sfär Silica HC D, Biotage), and purified by flash chromatography with a gradient mobile phase of dichloromethane (DCM) and methanol (MeOH). This gave 262 mg (36.9%) of DOPE-C3_α-2 as a yellow viscous oil.
¹H NMR (500 MHz, CD₃OD) δ: 0.88 (t, 12H), 1.21-1.38 (m, 40H), 1.60 (m, 8H), 2.01 (m, 8H), 2.31 (m, 4H), 2.80 (t, 2H), 3.33 (t, 4H), 3.96-4.18 (m, 7H), 4.42 (m, 1H), 4.80 (m, 1H), 5.22 (m, 1H), 5.33 (m, 4H).
MS (ESI): m/z calculated for C₄₉H₉₂NO₁₀P (M-H)⁻, 884.7; found, 884.4.

### (15) Synthesis of (R)-2,3-bis(oleoyloxy)propyl (2-((3-(heptan-4-yloxy)-3-oxopropyl)ammonio)ethyl) phosphate (DOPE-C4_α-3)

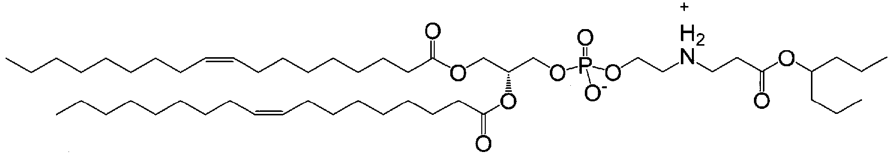

4-Heptanol (116.2 mg, 1.0 mmol) and TEA (209 µL, 1.50 mmol) were dissolved in anhydrous DCM (4 mL) and then were added with acryloyl chloride (80.8 µL, 1.0 mmol) dropwise in ice bath. The reaction mixture was stirred at ambient temperature for 2 hours. Solvent was removed in vacuo, and then the residue was suspended in hexane/AcOEt (9:1) and was passed through silica pad. The filtrate was concentrated in vacuo. The crude product (heptan-4-yl acrylate) was used for the next reaction without further purification.

DOPE (598.5 mg, 0.80 mmol), the crude product (heptan-4-yl acrylate) (165 mg), and TEA (209 µL, 1.50 mol) were dissolved in chloroform (4 mL) and were stirred at 60°C for 2 days under Ar atmosphere. The reaction mixture was loaded on normal-phase column (Sfär Silica HC D, Biotage), and purified by flash chromatography with a gradient mobile phase of dichloromethane (DCM) and methanol (MeOH). This gave 445 mg (60.9%) of DOPE-C4_α-3 as a yellow viscous oil.
¹H NMR (500 MHz, CD₃OD) δ: 0.88 (t, 12H), 1.21-1.38 (m, 40H), 1.50-1.63 (m, 8H), 2.01 (m, 8H), 2.31 (m, 4H), 2.80 (t, 2H), 3.33 (t, 4H), 3.96-4.18 (m, 7H), 4.42 (m, 1H), 4.95 (m, 1H), 5.22 (m, 1H), 5.33 (m, 4H).
MS (ESI): m/z calculated for C₅₁H₉₆NO₁₀P (M-H)⁻, 912.7; found, 912.6.

### [Test Examples]

### (1) Materials

A pH-sensitive cationic lipid, CL4F6, was synthesized according to a conventional method (see, for example, Patent Literature 2). Cholesterol (Chol) was purchased from SIGMA Aldrich. 1,2-Distearoyl-sn-glycero-3-phosphocholine (DSPC), 1,2-dioleoyl-sn-glycero-3-phosphoethanolamine (DOPE), 1-palmitoyl-2-oleoyl-sn-glycero-3-phosphocholine (POPC), and 1,2-dimirystoyl-rac-glycero-3-methoxypolyethyleneglycol-2000 (PEG-DMG) were obtained from NOF Corporation. 1,1'-Dioctadecyl-3,3,3',3'-tetramethylindocarbocyanine perchlorate (DiI), 1,1'-dioctadecyl-3,3,3',3'-tetramethylindotricarbocyanine iodide (DiR), and Ribogreen were purchased from Molecular Probes. 2-(p-Toluidino)-6-napthalene sulfonic acid (TNS) was obtained from Wako Chemicals. The fluorescein-labeled Lycopersicon esculentum (Tomato) lectin was purchased from Vector Laboratories. The Flue-encoding mRNA and the EGFP-encoding mRNA were purchased from Trilink BioTechnologies. Poly(A) was purchased from SIGMA Aldrich. A microfluidic device, an invasive lipid nanoparticle production (iLiNP) device, was fabricated in accordance with the disclosure of Non-patent Literature 7.

BALB/c mice (female, 4 weeks of age) were purchased from Japan SLC. The experimental protocols were reviewed and approved by the Hokkaido University Animal Care Committee in accordance with the guidelines for proper implementation of animal experiments.

### (2) Properties of mRNA-LNP containing DOPE-Cx

Ethanol solutions containing each phospholipid, CL4F6, Chol, and PEG2000-dimirystoylglycerol (PEG-DMG) at the molar ratio of 10:50:40:1.0 were prepared at the total lipid concentration of 16 mM. For fluorescent labeling of LNPs, 0.5 mol% of DiI or DiR was added to the above solutions. The firefly luciferase (Fluc)-encoding mRNA (TriLink) was dissolved in a 25 mM acetate buffer (pH 4.0). LNPs were prepared by mixing the lipids in ethanol and RNA in an aqueous solution using an iLiNP device at a total flow rate (TFR) of 0.5 mL/min and a flow rate ratio (FRR) (RNA flow rate/lipid flow rate) of 3. The nitrogen to phosphate (N/P) ratio was adjusted to 6. Syringe pumps (Harvard apparatus) were used to control the flow rate of the solutions. The resulting LNP solutions were then dialyzed for 2 hours or more at 4 °C against phosphate buffered saline without Ca²⁺ and Mg²⁺ (PBS(-)) using Spectra/Por 4 dialysis membranes (molecular weight cut-off 12,000-14,000 Da, Spectrum Laboratories). The LNP solutions were concentrated by ultrafiltration using an Amicon Ultra-15 unit (MWCO 100 kDa, Millipore) as needed.

The ζ-average particle size, polydispersity index (PDI), and ζ-potential of the LNPs were measured by means of a Zetasizer Nano ZS ZEN3600 instrument (Malvern Instruments). The encapsulation efficiency and total concentration of mRNA were measured by a Ribogreen assay according to a conventional method.

In addition, a TNS assay was conducted according to a conventional method. Briefly, 30 µM LNP lipid and 6 µM 2-(p-toluidino)naphthalene-6-sulfonic acid (TNS) were mixed in 50 µL of 20 mM citrate buffer, 20 mM sodium phosphate buffer, or 20 mM Tris-HCl buffer, containing 130 mM NaCl, at a pH ranging from 3.5 to 9.5. Fluorescence was measured by a multispectro microplate reader, Varioskan Flash set up with λex = 321 nm, λem = 447 nm at 37 °C. The pKa values were measured as the pH giving rise to half-maximal fluorescent intensity.

The results are shown in Tables 1 to 3. In the mRNA-LNP formulation containing DOPE-C4, C6, C8, C10, C12, or C18:1, no significant change in physical property values was observed as compared with the conventional mRNA-LNP formulation containing DSPC or DOPE. Also, in the mRNA-LNP formulation containing DOPE-C5_β1, C6_β2, C7_β3, C8_β4, or Cit, no significant change in physical property values was observed as compared with the conventional mRNA-LNP formulation containing DOPE. Further, in the mRNA-LNP formulations containing DOPE-C3_α2, C4_α3, or C8, no significant change in physical property values was observed mutually.

**Table 1: Properties of mRNA-LNP formulations**

| Phospholipid | ζ-average particle size (nm) | Number average particle size (nm) | PDI | ζ-potential (mV) | mRNA encapsulation rate % | pKa |
|---|---|---|---|---|---|---|
| DSPC | 75 | 56 | 0.08 | -3.6 | 95.7 | 6.20 |
| DOPE | 88 | 57 | 0.25 | -2.5 | 93.0 | 6.28 |
| DOPE-C4 | 87 | 58 | 0.17 | -5.0 | 91.2 | 6.30 |
| DOPE-C6 | 86 | 59 | 0.15 | -1.4 | 91.5 | 6.29 |
| DOPE-C8 | 94 | 62 | 0.21 | -5.6 | 92.6 | 6.31 |
| DOPE-C10 | 82 | 48 | 0.18 | -7.0 | 93.1 | 6.24 |
| DOPE-C12 | 100 | 59 | 0.25 | -6.3 | 94.2 | 6.29 |
| DOPE-C18:1 | 81 | 56 | 0.18 | -2.9 | 93.9 | 6.31 |

**Table 2: Properties of mRNA-LNP formulations**

| Phospholipid | ζ-average particle size (nm) | Number average particle size (nm) | PDI | ζ-potential (mV) | mRNA encapsulation rate % |
|---|---|---|---|---|---|
| DOPE | 95 | 67 | 0.13 | -2.9 | 92.8 |
| DOPE-C5_β1 | 94 | 60 | 0.21 | -6.4 | 95.9 |
| DOPE-C6_β2 | 94 | 73 | 0.07 | -5.0 | 95.7 |
| DOPE-C7_β3 | 79 | 61 | 0.08 | -9.6 | 96.0 |
| DOPE-C8_β4 | 112 | 71 | 0.21 | -5.8 | 96.3 |
| DOPE-Cit | 85 | 65 | 0.07 | -6.1 | 95.9 |

**Table 3: Properties of mRNA-LNP formulations**

| Phospholipid | ζ-average particle size (nm) | Number average particle size (nm) | PDI | ζ-potential (mV) | mRNA encapsulation rate % | pKa |
|---|---|---|---|---|---|---|
| DOPE-C3_α2 | 91 | 54 | 0.20 | -10.5 | 97.2 | 6.30 |
| DOPE-C4_α3 | 91 | 52 | 0.21 | -12.0 | 97.1 | 6.30 |
| DOPE-C8 | 80 | 55 | 0.17 | -12.8 | 97.6 | 6.29 |

### (3) mRNA introduction ability of DOPE-Cx-containing mRNA-LNP

The mRNA-LNP formulations prepared in the above item (2) were intravenously administered to BALB/c mice at a dose of 0.01 mg RNA/kg, and the livers were collected by a conventional method 6 hours later. The liver tissues were homogenized in a lysis buffer (Promega) using Micro Smash MS-100R (Tomy Seiko Co., Ltd.), and debris was removed by centrifugation. The supernatant was collected, and fluorescence was measured by a luminometer (Luminescencer-PSN, ATTO Corporation) using Luciferase Assay System (Promega). The protein concentration was determined using a BCA protein assay kit (Pierce). Fluc activity was expressed as relative light units (RLU) per 1 mg of protein.

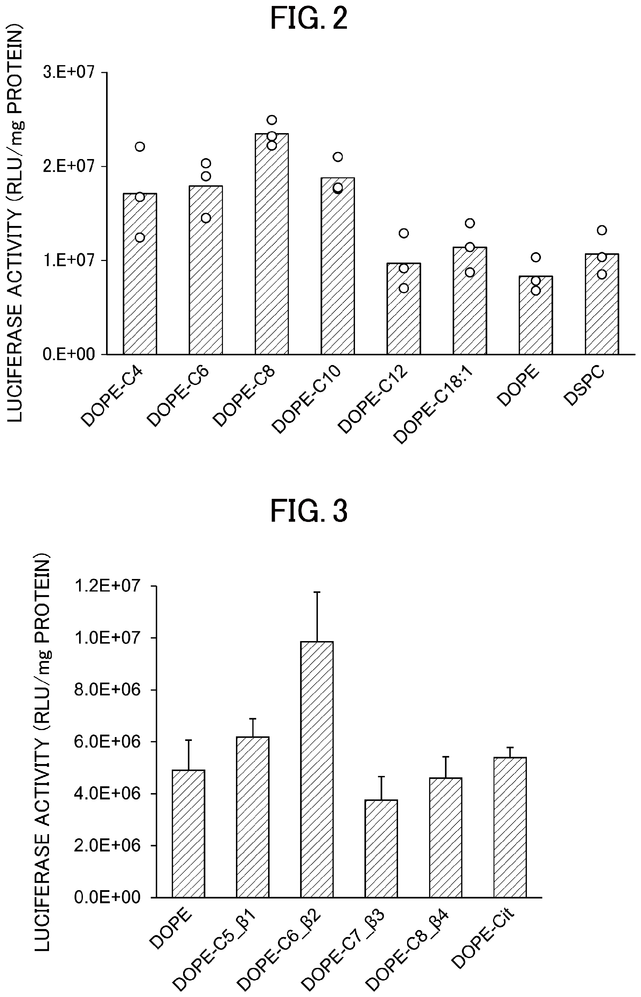

The results are shown in Fig. 2 (n=3). When the LNP containing DOPE-Cx was administered, luciferase activity equal to or higher than that in the case of administering the LNP containing DSPC or DOPE which is a conventional phospholipid was observed. In particular, the highest activity was shown when the LNP containing DOPE-C8 was administered.

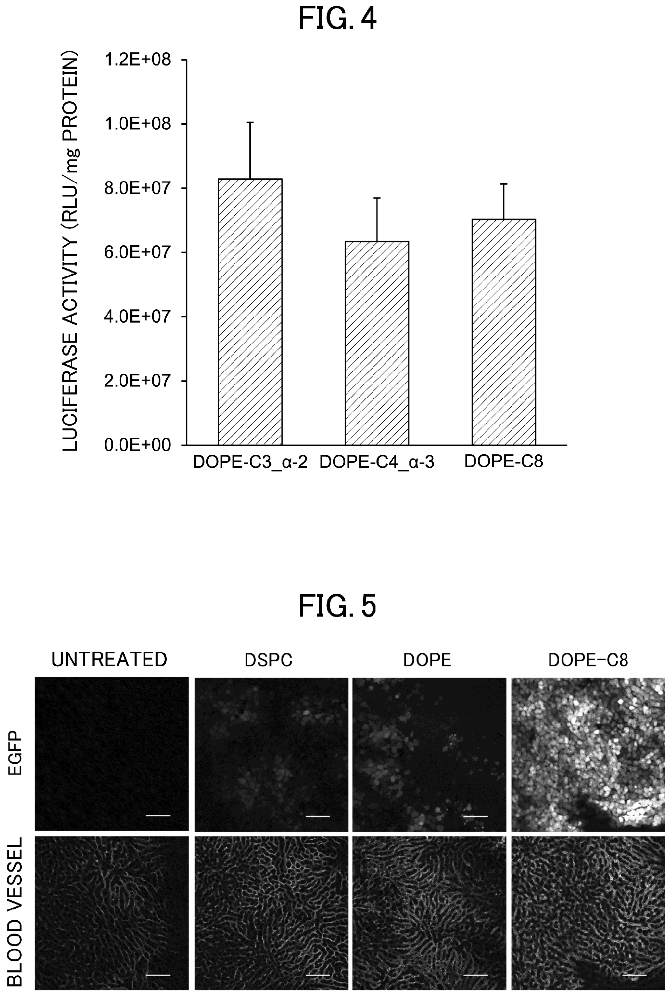

Similarly, the results are shown in Figs. 3 and 4 (n=3 except for DOPE-C8 in Fig. 4, n=4 for DOPE-C8 in Fig. 4). Similar to the results shown in Fig. 2, when the LNP containing DOPE-Cx was administered, luciferase activity equal to or higher than that in the case of administering the LNP containing DOPE which is a conventional phospholipid was observed. In particular, the highest activity was shown when the LNP containing DOPE-C6_β2 was administered.

Further, an mRNA-LNP formulation was prepared in the same manner as in the above item (2) except that mRNA encoding EGFP (TriLink) was used as mRNA, and it was intravenously administered to BALB/c mice at a dose of 1 mg RNA/kg. Then, 24 hours later, the liver was collected by a conventional method, and expression of EGFP was observed using a confocal laser scanning microscope (CLSM).

The results are shown in Fig. 5. When the LNP containing DOPE-C8 was administered, significantly higher expression of EGFP was observed as compared with the case of administering the LNP containing DSPC or DOPE which is a conventional phospholipid. And it was revealed that the gene expression occurred mainly in hepatocytes.

### (4) Membrane disruption ability of DOPE-Cx-containing LNP

In order to use as an index of endosomal escape efficiency of LNP, membrane disruption ability of LNP was evaluated using erythrocytes as a model biological membrane. Specifically, fresh erythrocytes (RBCs) were collected from BALB/c mice and suspended in a buffer solution (10 mM malic acid, 10 mM HEPES, 10 mM MES, and 120 mM NaCl) at pH 5.0, 6.0, or 7.4. The RBC suspension was mixed with the same amount of each LNP suspension (containing DOPE-C8, DOPE, or DSPC as phospholipid) and reacted at 37°C for 30 minutes. Then, the supernatant was collected, absorbance at 545 nm was measured, and hemoglobin leaked from the erythrocytes was quantified. As a positive control, a sample hemolyzed with 0.5 w/v% Triton X-100 was prepared, and as a negative control, a sample not containing LNP was prepared. The ratio of the absorbance of the test sample to the absorbance of the positive control was expressed as % hemolytic activity.

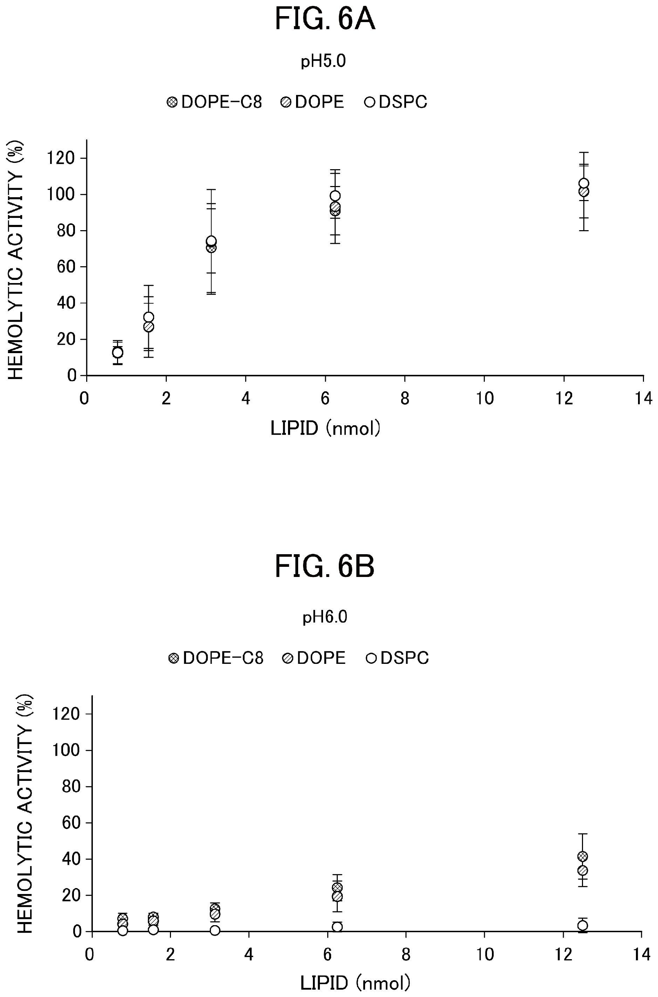

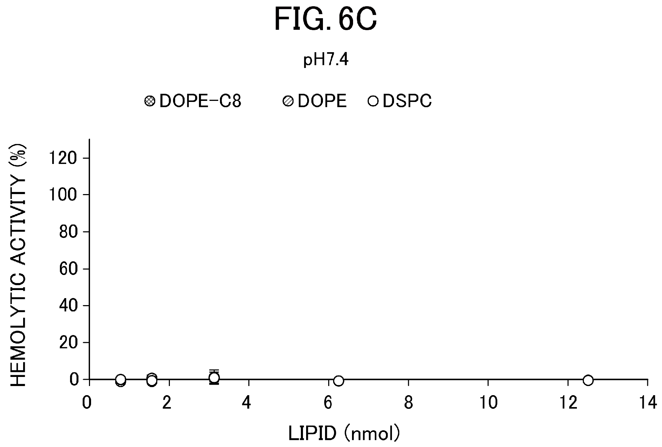

The results are shown in Figs. 6A to 6C. None of the LNPs induced membrane disruption at pH 7.4 (Fig. 6C), whereas they induced significant membrane disruption in a lipid amount-dependent manner at pH 5.0 (Fig. 6A). On the other hand, at pH 6.0, the LNP containing DOPE-C8 or DOPE showed higher membrane disruption activity than the LNP containing DSPC (Fig. 6B). Although this condition of pH 6.0 corresponds to the condition of early endosomes, it is not a sufficient pH for ionizable lipids such as CL4F6 to be positively charged. Even under such conditions, it was suggested that DOPE-C8 can improve endosomal escape efficiency.

### (5) Evaluation of lipid polymorphism by ³¹P NMR method

In order to examine the interaction between DOPE-Cx and PC and characteristics thereof, lipid polymorphism of the lipid suspension was evaluated by a ³¹P NMR method. Since the rotational motion of lipid molecules in a lamellar phase which is a planar structure and a (inverted) hexagonal phase which is a tubular structure receives different restrictions respectively, a specific ³¹P NMR spectrum is provided by chemical shift anisotropy. Specifically, the lamellar phase produces a broad high-field peak, and the (inverted) hexagonal phase produces a broad low-field peak. On the other hand, a (inverted) cubic phase and a (inverted) micellar phase show a sharp peak centered at 0 ppm without generating chemical shift anisotropy because they are three-dimensionally equivalent structures in a minute space.

First, DOPE or DOPE-C8 and 1-palmitoyl-2-oleoyl-sn-glycero-3-phosphocholine (POPC) were mixed at a predetermined ratio in 400 µL of chloroform (total lipid 16 µmol), and the solvent was removed by a rotary evaporator to form a lipid membrane. The lipid membrane was hydrated with 500 µL of 20 mM HEPES buffer (pH 7.4, 130 mM NaCl) at 60°C. Then, the lipid dispersion was transferred to an NMR tube, and a ³¹P NMR spectrum at room temperature was measured by a conventional method using an ECX 400P spectrometer (JEOL Ltd.).

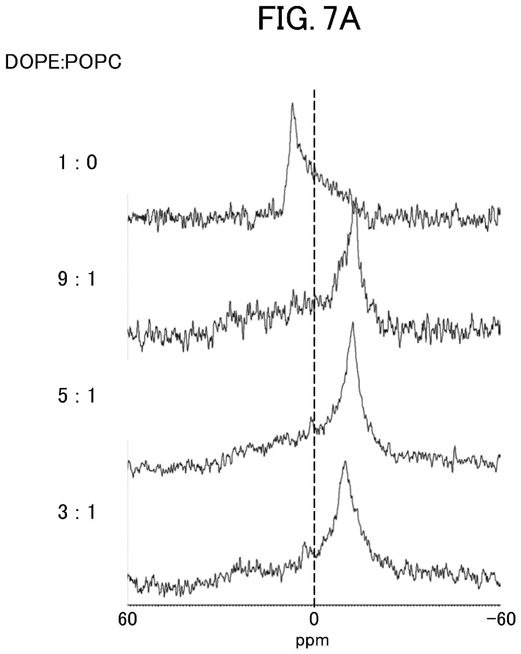

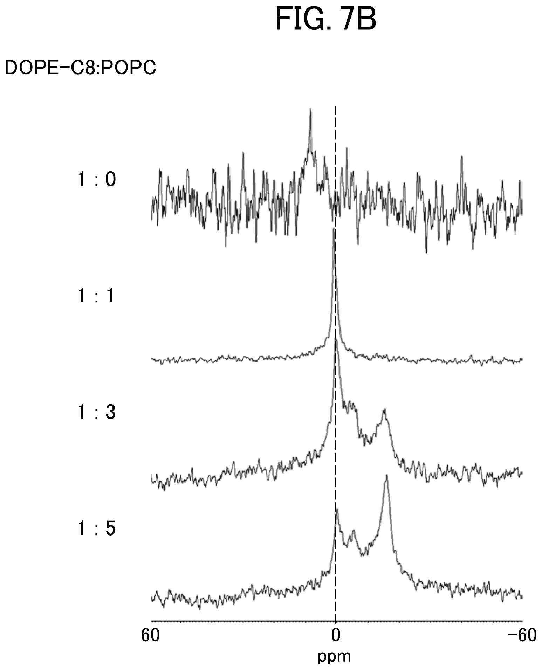

The results are shown in Figs. 7A and 7B. With DOPE alone (DOPE:POPC=1:0), a spectrum derived from the inverted hexagonal phase was obtained as reported previously (Fig. 7A). And at the time when POPC was mixed at 10% in molar ratio (DOPE:POPC=9:1), the peak derived from the inverted hexagonal phase disappeared and transition to the lamellar phase occurred, so inhibition of phase transition by PC was recognized (Fig. 7A). On the other hand, in the case of DOPE-C8, a spectrum derived from the inverted hexagonal phase was obtained although noise was large when used alone (DOPE-C8:POPC=1:0), and transition to the lamellar phase was not seen even when mixed with POPC up to an equivalent amount (DOPE-C8:POPC=1:1), and a sharp peak centered at 0 ppm was obtained (Fig. 7B). And when the POPC content was further increased, the peak derived from the lamellar phase became stronger in a POPC content-dependent manner, while the peak centered at 0 ppm also remained (Fig. 7B).

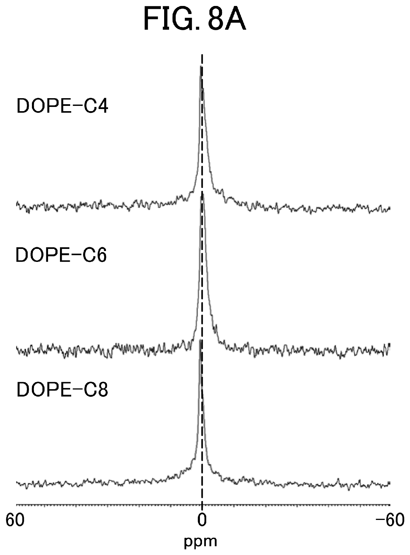

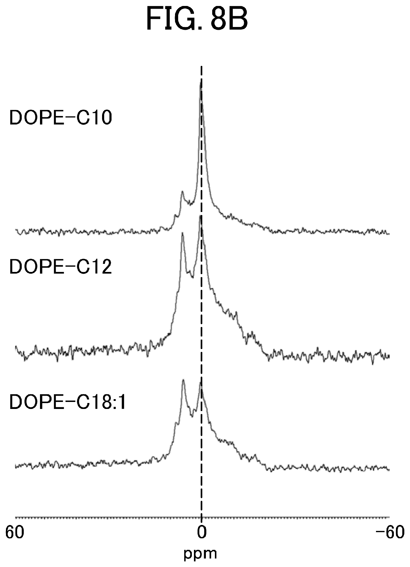

Next, regarding various DOPE-Cx, ³¹P NMR spectra when mixed with POPC at a molar ratio of 1:1 were measured and compared. The results are shown in Figs. 8A and 8B. Even when any DOPE-Cx was used, transition to the lamellar phase was not observed. In particular, when DOPE-C4, DOPE-C6, or DOPE-C8 was used, a similar sharp peak centered at 0 ppm was obtained. When DOPE-C10, C12, or C18:1 was used, the ratio derived from the inverted hexagonal phase increased in a manner dependent on the number of carbon atoms thereof.

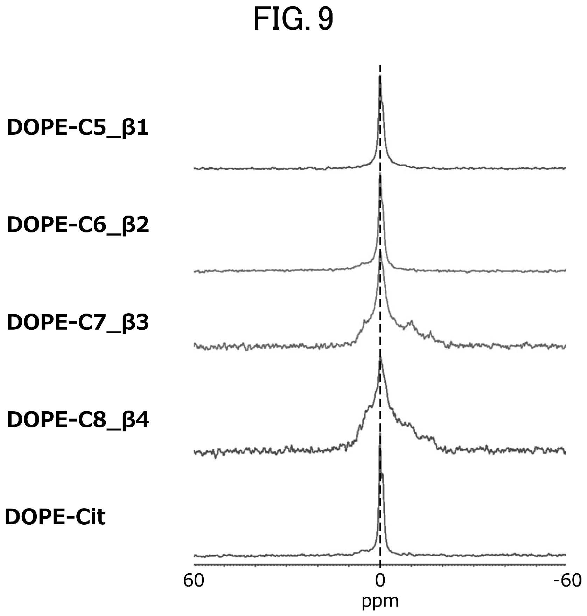

Similarly, the results are shown in Fig. 9. Even when any DOPE-Cx was used, transition to the lamellar phase was not observed. In particular, when DOPE-C5_β1, DOPE-C6_β2, or DOPE-Cit was used, a similar sharp peak centered at 0 ppm was obtained. When DOPE-C7_β3 or DOPE-C8_β4 was used, the ratio derived from the inverted hexagonal phase increased in a manner dependent on the number of carbon atoms thereof.

### (6) Evaluation of lipid polymorphism by SAXS method

In order to identify the phase of the lipid suspension obtained by mixing DOPE-C8 and POPC at a molar ratio of 1:1, measurement by a small-angle X-ray scattering (SAXS) method was performed by a conventional method using a NANO-Viewer apparatus (Rigaku Corporation).

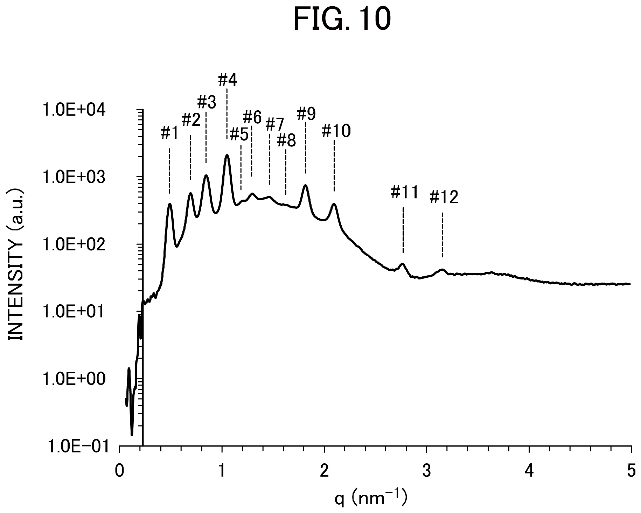

The results are shown in Fig. 10 and Table 4. Since peaks of 1/√2 (#2) and 1/√6 (#5) were observed, it was suggested that the isotropic peak observed by the ³¹P NMR method was derived from an inverse bicontinuous cubic (Pm3m, Im3m) phase. In addition, since 1/√7 (#6) was observed, it was considered high likely to be an Im3m type inverse bicontinuous cubic phase.

**Table 4: Results of SAXS method**

| # | 2θ (deg) | q (nm⁻¹) | Half Width | d (nm) | Ratio of d values |
|---|---|---|---|---|---|
| 1 | 0.685 | 0.49 | 0.098 | 12.9 | 1.000 |
| 2 | 0.971 | 0.69 | 0.135 | 9.1 | 0.705 |
| 3 | 1.184 | 0.84 | 0.116 | 7.5 | 0.579 |
| 4 | 1.471 | 1.05 | 0.084 | 6.0 | 0.466 |
| 5 | 1.678 | 1.19 | 0.106 | 5.3 | 0.408 |
| 6 10 | 1.810 | 1.29 | 0.151 | 4.9 | 0.378 |
| 7 | 2.046 | 1.46 | 1.422 | 4.3 | |
| 8 | 2.272 | 1.62 | 0.166 | 3.9 | |
| 9 | 2.547 | 1.81 | 0.230 | 3.5 | |
| | 2.943 | 2.10 | 0.097 | 3.0 | |
| 11 | 3.882 | 2.76 | 0.115 | 2.3 | |
| 12 | 4.428 | 3.15 | 0.118 | 2.0 | |

It is known that virus-derived membrane fusion peptides (such as influenza hemagglutinin peptide and feline leukemia virus fusion peptide) induce membrane fusion via induction to an inverse bicontinuous cubic phase (see Siegel DP et al., Biochim Biophys Acta 1468, 87-98 (2000) and Malcolm JM et al., FEBS Lett 461, 178-182 (1999), etc.), and the importance of ability to induce the inverse bicontinuous cubic phase in a nucleic acid introduction strategy mediated by membrane fusion is suggested. Therefore, it was suggested that DOPE-Cx such as DOPE-C8 contributes to efficient substance delivery by the effect of promoting induction to the inverse bicontinuous cubic phase.

From the above, it was found that the compound represented by the above formula (I) is useful as a constituent lipid of a lipid nanoparticle enabling efficient substance delivery into a cell.

## Claims

1. A compound represented by formula (I): 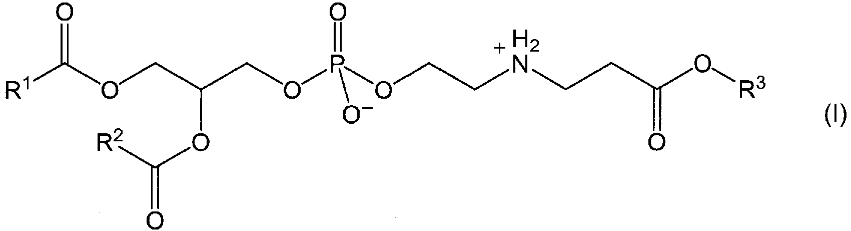 wherein
R¹ and R² are each independently a chain hydrocarbon group having 10 to 24 carbon atoms, and
R³ is a chain hydrocarbon group having 1 to 22 carbon atoms.

2. The compound according to claim 1, wherein R¹ and/or R² is an unsaturated hydrocarbon group.

3. The compound according to claim 1, wherein R¹ and/or R² is an alkenyl group, and/or
R³ is an alkyl group or an alkenyl group.

4. The compound according to claim 1, wherein the number of carbon atoms of R¹ is 12 to 22, and/or
the number of carbon atoms of R² is 12 to 22, and/or
the number of carbon atoms of R³ is 2 to 20.

5. The compound according to claim 1, wherein R¹ and/or R² is 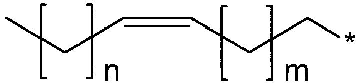 wherein
an asterisk represents a binding site,
n is an integer of 5 to 9, m is an integer of 2 to 8, provided that n+m is 12 to 16,
and/or
R³ is 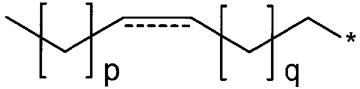 wherein
an asterisk represents a binding site,
p is an integer of 0 to 16, q is an integer of 0 to 16, provided that p+q is 0 to 16, and
a double line composed of a solid line and a dashed line means a single bond or a double bond.

6. The compound according to claim 1, wherein R³ is 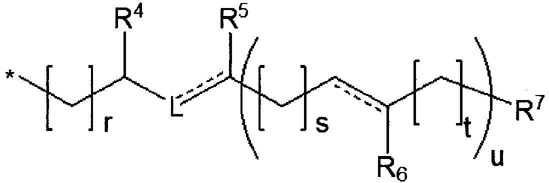 wherein
an asterisk represents a binding site,
R⁴ is a hydrogen atom or an alkyl group having 1 to 6 carbon atoms,
R⁵ is a hydrogen atom or an alkyl group having 1 to 6 carbon atoms,
R⁶ is a hydrogen atom or an alkyl group having 1 to 6 carbon atoms,
provided that at least one of R⁴, R⁵, and R⁶ is an alkyl group having 1 to 6 carbon atoms,
L is a single bond, an alkylene group having 1 to 4 carbon atoms, -CH=CH(CH₂)₂-, -CH₂CH=CHCH₂-, or -CH=CHCH₂-,
R⁷ is a hydrogen atom or a methyl group,
r is an integer of 0 to 4,
s is an integer of 0 to 4,
t is an integer of 0 to 4,
u is 0 or 1,
double lines composed of a solid line and a dashed line each independently mean a single bond or a double bond.

7. The compound according to claim 1, having any of the following structures: 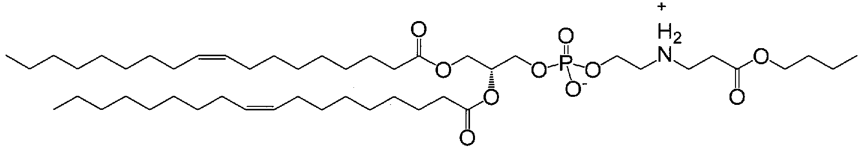 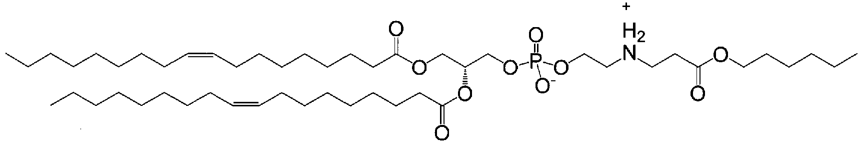 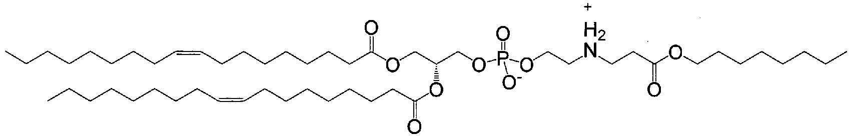 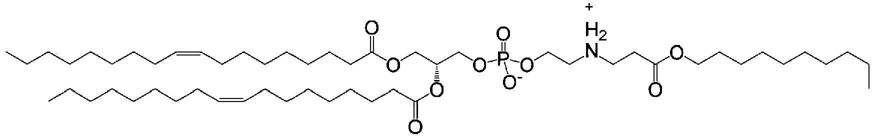 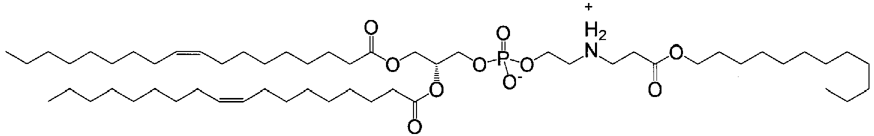 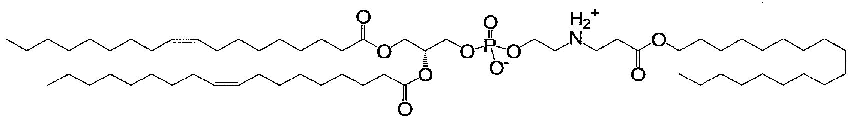 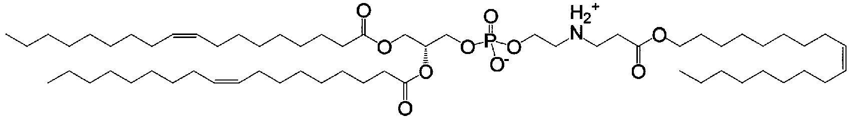 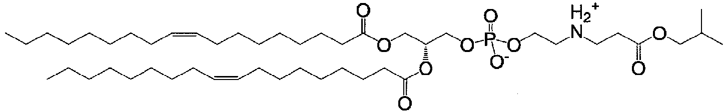 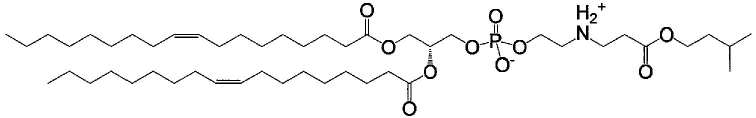 and

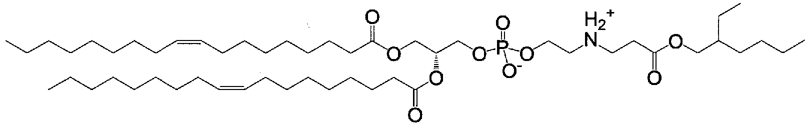

8. The compound according to claim 1, having any of the following structures: 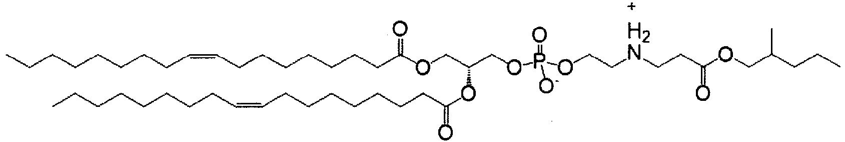 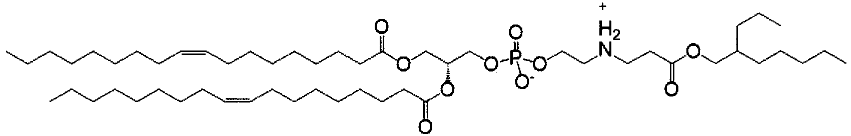 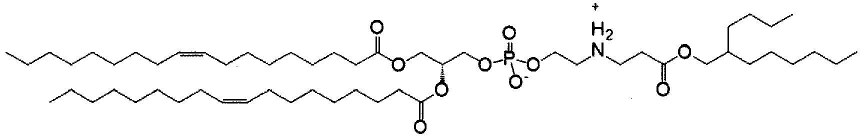 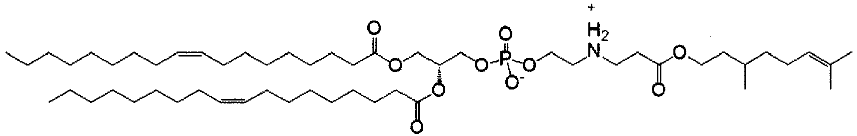 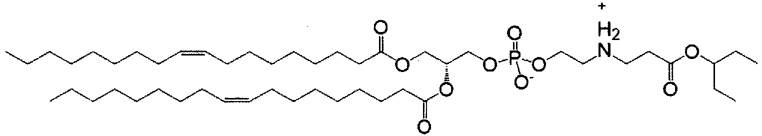 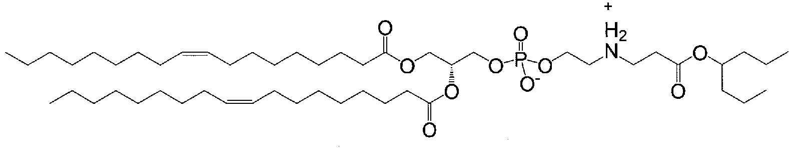 and

9. A method for producing the compound according to any one of claims 1 to 8, comprising a step of adding, to a compound represented by formula (II): 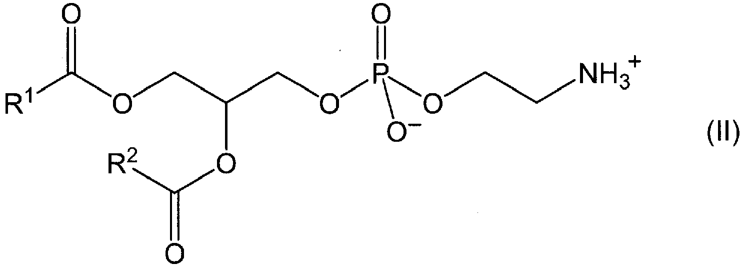
wherein R¹ and R² are as defined in claim 1,
a compound represented by formula (III): 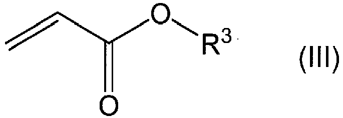
wherein R³ is as defined in claim 1.

10. A lipid nanoparticle comprising the compound according to any one of claims 1 to 8.

11. The lipid nanoparticle according to claim 10, further comprising a sterol and/or a polyalkylene glycol-modified lipid.

12. The lipid nanoparticle according to claim 10, further comprising a pH-sensitive cationic lipid having a pKa of 6.6 or less.

13. The lipid nanoparticle according to claim 10, further comprising a nucleic acid.

14. The lipid nanoparticle according to claim 13, wherein the nucleic acid comprises siRNA, mRNA, and/or plasmid DNA.

15. A composition comprising the lipid nanoparticle according to claim 10.

16. The composition according to claim 15, wherein the lipid nanoparticle comprises a nucleic acid.

17. The composition according to claim 15, for expressing a foreign gene and/or for use in gene therapy.
